(19) Europäisches Patentamt
European Patent Office
Office européen des brevets

(11) EP 1 337 532 B1

(12) **EUROPEAN PATENT SPECIFICATION**

(45) Date of publication and mention of the grant of the patent:
**11.05.2005 Bulletin 2005/19**

(21) Application number: **01980839.3**

(22) Date of filing: **12.11.2001**

(51) Int Cl.[7]: **C07D 451/02**, C07C 333/20, C07F 13/00, C07F 1/08, A61K 51/04

(86) International application number:
**PCT/IB2001/002141**

(87) International publication number:
**WO 2002/044175 (06.06.2002 Gazette 2002/23)**

(54) **COMPOUND FOR CHELATING A METAL, RADIOPHARMACEUTICAL, MANUFACTURING PROCESS THEREFOR, AND DIAGNOSTIC KIT**

METALLCHELATVERBINDUNG, RADIOPHARMAZEUTISCHES PRODUKT, VERFAHREN ZU SEINER HERSTELLUNG UND DIAGNOSE-KIT

COMPOSE POUR CHELATER UN METAL, PRODUIT RADIOPHARMACEUTIQUE, PROCEDE DE FABRICATION DE CE DERNIER ET KIT DE DIAGNOSTIC

(84) Designated Contracting States:
**AT BE CH CY DE DK ES FI FR GB GR IE IT LI LU MC NL PT SE TR**

(30) Priority: **29.11.2000 FR 0015416**

(43) Date of publication of application:
**27.08.2003 Bulletin 2003/35**

(73) Proprietor: **SCHERING AKTIENGESELLSCHAFT
13342 Berlin (DE)**

(72) Inventors:
- **MAUCLAIRE, Laurent
F-75013 Paris (FR)**
- **BERTHOMMIER, Eric
F-92350 Le Plessis Robinson (FR)**

(74) Representative: **Poulin, Gérard et al
Société BREVATOME
3, rue du Docteur Lancereaux
75008 Paris (FR)**

(56) References cited:
**WO-A-97/14445**

- **B.K. MADRAS, ET AL.: "Technepine: A high-affinity 99m-technetium probe to label the dopamine transporter in brain by SPECT imaging" SYNAPSE, vol. 22, no. 3, 1996, pages 239-246, XP002100841 Wiley and Sons, Chichester, GB ISSN: 0887-4476**
- **M.-P. KUNG, ET AL.: "[99mTc]TRODAT-1: a novel technetium-99m complex as a dopamine transporter imaging agent" EUROPEAN JOURNAL OF NUCLEAR MEDICINE, vol. 24, no. 4, April 1997 (1997-04), pages 372-380, XP001010628 Springer-Verlag, Berlin, DE ISSN: 0340-6997**

EP 1 337 532 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

### Technical field of the invention

The present invention relates to a ligand for chelating a metal or a metal complex, to a radiopharmaceutical, to a manufacturing process therefor and to a diagnostic kit.

The ligand may be used to manufacture a diagnostic product or a medicinal product.

The metal may be a transition metal chosen, for example, from Tc, Ru, Co, Cu, Pt, Fe, Os, Ir, Re, Cr, Mo, Mn, Ni, Rh, Pd, Nb, Sm and Ta or an isotope thereof.

The metal complex may be, for example, a nitride complex of radioactive transition metals which may be used as radiopharmaceutical products for diagnosis or therapy.

Among the complexes which may be used for diagnosis, mention may be made in particular of technetium 99m complexes.

Radiopharmaceutical products using the $^{99m}$Tc radionucleide are very useful in nuclear medicine for diagnosis on account of its physical and chemical characteristics. Technetium complexes which may be used for the present invention are described, for example, by E. DEUTSCH et al. in: Progr. Inorg. Chem. (Australia), vol. 30, pp. 76-106, 1983, and preparation processes are described in J. Baldas et al. in J. Chem. Soc. Dalton Trans 1981, pp. 1798-1801; in Int. Appl. Radiot. Isot. 36 (1985), pp. 133-139, in international patent application WO 85/03063 and in patent applications EP-A-537 242 and EP-A-0 403 524.

Documents WO 97/14445 and European Journal of Nuclear Medicine, vol. 24, n°4, 1997, pp.372-380 (same authors) disclose tropane-based derivatives that are specific for central nervous system receptors. The derivatives are usable as imaging agents for the central nervous system (CNS). The derivatives comprises an $N_2S_2$ ligand (bis-aminoethanethiol, i.e. two -NS groups) in which the ligand complexes with either the technetium or rhenium, or "three plus one complexes" (three -NS groups) where a tropane thiolate and an aminobisethanethiolate ligand are involved in complexation. This document does not disclose nor suggest a ligand having a bis-dithiocarbamate structure, i.e. a $C_2S_4$ ligand, including an amide group, for chelating a metal or a metal complex.

Document Synapse, vol.22, n°3, 1996, pages 239-246 discloses a probe having a high affinity for $^{99m}$technetium to label the dopamine transporter in brain by single photon emission computed tomography (SPECT) imaging. The probe is a phenyltropane having a $N_2S_2$ ligand (two -NS groups). This document does not disclose nor suggest a ligand having a bis-dithiocarbamate structure, i.e. a $C_2S_4$ ligand, including an amide group, for chelating a metal or a metal complex.

The complexes which may be used for therapy may be, for example, rhenium complexes.

Copper or an isotope thereof is useful for the present invention, for example for labelling antibodies or peptides, for diagnosis and especially for therapy ($^{67}$Cu, $^{64}$Cu).

### Description of the invention

The ligand for chelating a metal or a metal complex of the present invention is characterized in that it consists of a bis-dithiocarbamate structure (F) having the following formula:

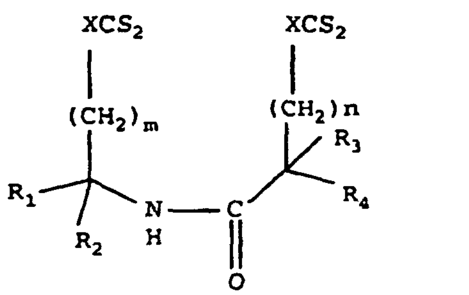

(F)

in which n and m are integers such that $5 \leq m+n \leq 10$, X is chosen independently from S and NH,
$R_1$, $R_2$, $R_3$ and $R_4$ are chosen independently from H and an organic function chosen from $-COOR_5$, $NR_5R_6$ and $-CH_2OR_5$ in which $R_5$ and $R_6$, when it is present, are chosen independently from a hydrogen; an amino acid; a peptide; a protein; an organic function; a group chosen from alkoxycarbonyl or aryloxycarbonyl ($-COOR^7$), carboxyl (-COOH), acyloxy ($-O_2R^7$), carbamoyl ($-CONR^7$), cyano (-CN), alkylcarbonyl, alkylarylcarbonyl, arylcarbonyl, arylalkylcarbonyl, hydroxyl (-OH), amino ($NR^7$), halogen, allyl, alkoxy ($-OR^7$), S-alkyl and S-aryl, $R^7$ representing a $C_1$ to $C_{10}$ alkyl or aryl group; an organic molecule chosen from (i) an optionally substituted alkyl, acyl, aryl or alkyne group, (ii) a saturated or unsaturated, optionally substituted or aromatic carbon-based ring or (iii) a saturated or unsaturated, optionally substituted or aromatic heterocycle, these groups and rings (i), (ii) and (iii) possibly being substituted with substituted phenyl groups, substituted aromatic groups or alkoxycarbonyl or aryloxycarbonyl (-$COOR^8$), carboxyl (-COOH), acyloxy (-$O_2R^8$), carbamoyl (-$CONR^8$), alkylcarbonyl, alkylarylcarbonyl, arylcarbonyl, arylalkylcarbonyl, hydroxyl (-OH), amino ($NR^8$), halogen, allyl, alkoxy (-$OR^8$), S-alkyl or S-aryl groups, $R^8$ representing a $C_1$ to $C_{10}$ alkyl or aryl group; a monoclonal antibody; a hormone; and a pharmaceutically acceptable vector.

n and m are natural integers. Thus, m and n may be, independently, 0, 1, 2, 3, 4, 5, 6, 7, 8, 9 or 10, provided that 5≤m+n≤10. For example, for m=0, n may be 5, 6, 7, 8, 9 or 10, and for m=1, n may be 4, 5, 6, 7, 8 or 9. According to the invention, the functions $XCS_2$ are in fact separated by a peptide bond and a number of carbon atoms greater than or equal to 7 and less than or equal to 10.

The present invention provides a novel system for complexing a metal or a metal complex which may be linked to any molecule or biomolecule. This system thus has numerous applications, especially for diagnosis and therapy.

According to the invention, the bis-dithiocarbamate compound may consist of a structure whose formula is chosen from formulae (I), (II), (III) and (IV) below:

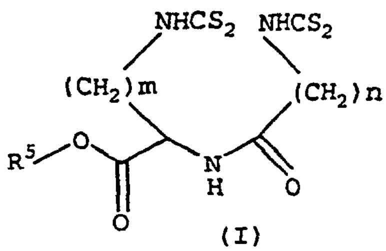

(I)

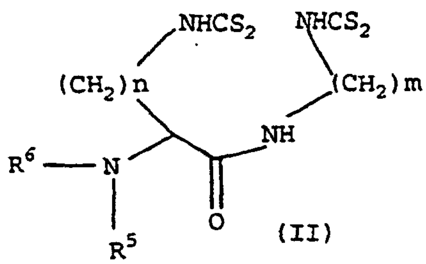

(II)

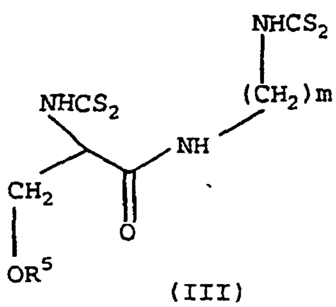

(III)

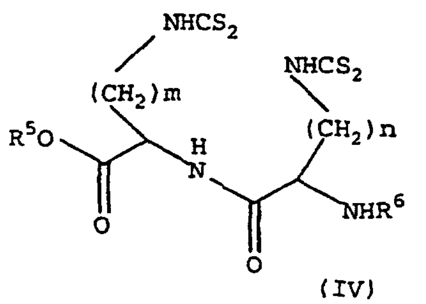

(IV)

in which n and $R^5$, and m and $R^6$, when they are present, are as defined above.

According to the invention, $R^5$ may be chosen from H, $CH_3$, a tropane derivative or a compound of formula:

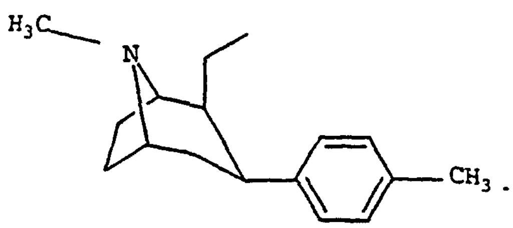

When $R^5$ is a tropane derivative, it may be of formula (G) below:

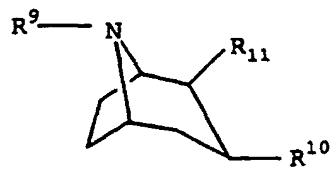

(G)

in which one of the radicals $R^9$, $R^{10}$ and $R^{11}$ is a compound of formula F, the other radicals being chosen independently from a hydrogen; an organic function; a group chosen from alkoxycarbonyl or aryloxycarbonyl (-$COOR^7$), carboxyl (-COOH), acyloxy (-$O_2R^7$), carbamoyl (-$CONR^7$), cyano (-CN), alkylcarbonyl, alkylarylcarbonyl, arylcarbonyl, arylalkylcarbonyl, hydroxyl (-OH), amino ($NR^7$), halogen, allyl, alkoxy (-$OR^7$), S-alkyl and S-aryl, $R^7$ representing a $C_1$ to $C_{10}$ alkyl or aryl group; an organic molecule chosen from (i) an optionally substituted alkyl, acyl, aryl or alkyne group, (ii) a saturated or unsaturated, optionally substituted or aromatic carbon-based ring or (iii) a saturated or unsaturated, optionally substituted or aromatic heterocycle, these groups and rings (i), (ii) and (iii) possibly being substituted with substituted phenyl groups, substituted aromatic groups or alkoxycarbonyl or aryloxycarbonyl (-$COOR^8$), carboxyl (-COOH), acyloxy (-$O_2R^8$), carbamoyl (-$CONR^8$), alkylcarbonyl, alkylarylcarbonyl, arylcarbonyl, arylalkylcarbonyl, hydroxyl (-OH), amino ($NR^8$), halogen, allyl, alkoxy (-$OR^8$), S-alkyl or S-aryl groups, $R^8$ representing a $C_1$ à $C_{10}$ alkyl or aryl group.

The ligand of the present invention comprising a tropane derivative may be used, for example, in the diagnosis of Parkinson's disease.

One example of a ligand according to the present invention may consist of a structure of formula (F) in which $R^1$, $R^3$ and $R^4$ =H and $R^2$ is $NR^5R^6$, in which $R^5$=H and $R^6$ is chosen from:

H or

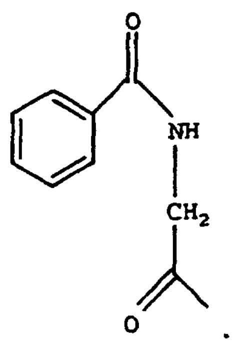

The ligands in which $R_6$ =

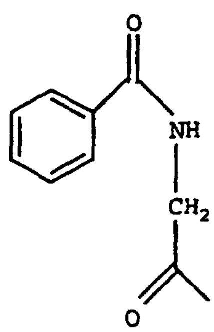

may be used, for example, for the diagnostic study of renal function or the therapy of renal pathologies.

The present invention also provides a chelation product (chelate) consisting of a ligand according to the present invention, and of a metal or a metal complex. Examples of metals and metal complexes have been described above.

For example, the metal may be chosen from copper, a copper isotope and a transition metal. This may be useful, for example, as a radiopharmaceutical for therapy or diagnosis.

For example, the metal complex may be TcN or ReN. This product may then be used as a radiopharmaceutical.

The present invention also relates to the use of a ligand or a chelate according to the present invention for manufacturing a medicinal product or a diagnostic product, for example a radiopharmaceutical for therapy or diagnosis. The radiopharmaceutical may be, for example, a radiopharmaceutical for visualizing the uptake of dopamine or serotonin. Such a radiopharmaceutical may be useful for diagnosing neurodegenerative diseases, for example Parkinson's disease.

The present invention also provides a process for manufacturing a bis-dithiocarbamate compound according to the invention, comprising, successively:

- a step of protecting the XH functions of the compounds of formulae (V) and (VI) below:

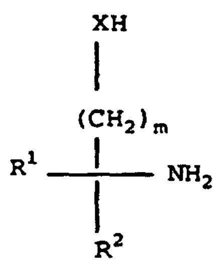

(V)

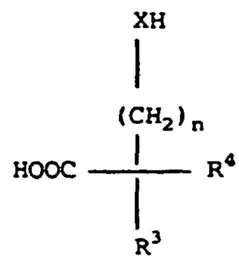

(VI)

in which $R^1$, $R^2$, $R^3$, $R^4$, X, m and n are as defined in the above claim,

- a step of activating the -COOH function of compound (VI),
- a step of linking the compound of formula (V) and compound (VI) via the activated carboxyl function of compound (VI),
- a step of deprotecting the XH functions, and
- a step of reacting the deprotected XH functions with $CS_2$ to form a bis-dithiocarbamate compound according to the present invention.

One of the starting compounds comprises an acid function and the other an amine function to form the peptide bond which links these compounds. In addition, each of the compounds must comprise at least one free $NH_2$ or SH function to attach the $CS_2$.

The aim of the step for protecting the XH functions of compounds (V) and (VI) is to protect these functions against the reagents used to activate the -COOH function of compound (VI), and to link compound (V) with the activated compound (VI). It is performed by means of conventional reagents known to those skilled in the art for protecting functions X, with X=S or NH. Examples are given below.

The other steps may each also be performed by processes known to those skilled in the art. Examples are given below to illustrate the present invention.

The base of the ligand of the present invention may be, for example, a combination of two natural or unnatural amino acids containing two amine functions in ε-terminal positions and, on axial chains, a function which will serve to functionalize the vector molecule for use in radiopharmacy. The amine functions in ε-terminal positions are modified into dithiocarbamate functions et serve to complex the metal, also known as the metallic core.

The present invention also relates to a process for manufacturing a ligand according to the present invention, the said process comprising a process for manufacturing a bis-dithiocarbamate compound according to the invention, and also comprising a step of attaching a radical $R^5$ and optionally $R^6$ to this bis-dithiocarbamide structure or to an intermediate product in its manufacture to obtain a ligand according to the invention as defined above.

The present invention thus provides a family of complexing agents which attach a metal, for example a radioelement, on the one hand, and which, by virtue of their functionalization, may be linked to a vector molecule either

via a final synthesis (linking of radicals $R^5$ or $R^6$), or during the synthesis of a vector molecule or a peptide.

The ligand for chelating a metal or a metal complex of the present invention may, for example, on the one hand, attach a radioelement, and, on the other hand, be linked to a vector molecule either via a final synthesis, or during the synthesis of a vector molecule or a peptide.

The present invention also relates to a process for manufacturing a ligand according to the invention, the said process comprising:

- a step of reacting two ε-$NH_2$ functions of two contiguous amino acids of a precursor molecule of the ligand according to Claim 1 or 2 with $CS_2$ so as to form a ligand according to Claim 1 or 2,

the precursor molecule constituting the radical $R^5$ and optionally the radical $R^6$.

The precursor molecule may be, for example, in the form of the compounds (V) and (VI) described above linked via a peptide bond formed between the $NH_2$ and COOH side functions.

The ligand of the present invention may thus also be obtained, for example, by dithiocarbamate (DTC) labelling of two contiguous natural or unnatural amino acids, on the $NH_2$ functions of the side chains, or on an $NH_2$ function of a side chain and an N-terminal $NH_2$ function in the case of a dipeptide or of a reaction at the N-terminal end of a peptide or a protein. It may also be obtained by DTC-labelling of an organic molecule comprising a peptide bond and two amine functions separated by at least seven carbons.

The process of the present invention may be used, for example, to manufacture a chelate according to the invention defined above, comprising the manufacture of a bis-dithiocarbamate compound according to the invention according to a manufacturing process of the present invention, and a reaction for complexing a metal or a metal complex via the said bis-dithiocarbamate compound manufactured.

The metal or the metal complex may be as defined above.

The present invention also provides a diagnostic kit comprising a ligand according to the present invention.

Other characteristics and advantages of the invention will also emerge on reading the examples which follow.

## EXAMPLES

### A) EXAMPLES OF THE PREPARATION OF BIS-DITHIOCARBAMATES ACCORDING TO THE PRESENT INVENTION FROM DIPEPTIDES

Example 1 : Preparation of bis-dithiocarbamates from the following sequences: lysine-lysines (1), alanine-lysine (2) and glycine-lysine (3)

Each diamino molecule (0.5 mmol) is suspended in 10 ml of ethanol and sodium hydroxide pellets (0.08 g ; 2 mmol) are added along with the minimum amount of water required to dissolve the amino molecule. The mixture is left stirring for 1 hour and the water/ethanol mixture is then evaporated off under reduced pressure at low temperature. The oil obtained is taken up in alcohol, 3 sodium hydroxide pellets are added and an excess of pure carbon disulphide $CS_2$ is then introduced dropwise. A yellow coloration appears after half an hour, and the copper sulphate test is positive. This test was performed by placing a drop of reaction mixture on a silica plate and then, on this drop, a drop of copper sulphate in water: a brown spot is observed if the bis-dithiocarbamate is formed. After stirring for 4 hours, the solvent is evaporated off to dryness, giving a yellow paste.

HPLC analysis : C18-5 μm-25 cm-YMC column; flow rate 1 ml/minute; UV detection at 300 nm; eluents A=water; B=methanol; gradient 0 to 5 minutes 0% of B - from 5 to 20 minutes 0 to 100% of B - from 20 to 25 minutes 100% of B; 25 to 25.1 minutes 100 to 0% of B - 25.1 to 30 minutes 0% of B. Purity of the synthesized products greater than 95% in the three cases.

Example 2: Radiolabelling of the lysine-lysines (DTClys-lysDTC): compound (4); alanine-lysine (DTCala-lysDTC): compound (5) and glycine-lysine (DTCgly-lysDTC): compound (6) bis-dithiocarbamates

Protocol for radiolabelling with $^{99}Tc$ :

a-intermediate solution: a lyophilisate containing hydrazine (SDH) 5 mg, 1,2-propanediamino-N,N,N',N'-tetraacetic acid (PDTA) 5 mg, 10 μg of stannic chloride is taken up in 1 ml of injection-grade water. 1 ml of $^{99}TcO_4^-$ is added. The mixture is left to act for 30 minutes.

b-exchange reaction: 1.8 mg of bis-dithiocarbamate dissolved in a 0.1 M pH 7.4 phosphate buffer are mixed with 0.5 ml of the preceding intermediate solution. The mixture is left to act for 2 hours.

The reactions are analysed by HPLC. The results of these analyses are given in Table I below:

| Product | Detection | Number of peaks |
|---|---|---|
| (DTCgly-lysDTC) | radioactive | 9 peaks |
| (DTCala-lysDTC) | radioactive | 6 peaks |
| (DTClys-lysDTC) | radioactive | 1 peak |

The first two bis-dithiocarbamates react intermolecularly whereas the (DTCgly-lysDTC) bis-dithiocarbamate reacts via an intramolecular reaction.

**B) EXAMPLES OF STEPS FOR PROTECTING THE XH FUNCTIONS OF COMPOUNDS OF FORMULAE V AND VI ACCORDING TO THE PROCESS OF THE INVENTION**

Example 3 : Synthesis of N-trifluoroacetyl-5-amino-valeric acid: compound (7)

1.6 equivalents of S-ethyl trifluorothioacetate (16 mmol; 2 ml) are added dropwise, using a syringe, to a solution of 5-aminovaleric acid (5a) (10 mmol; 1.17 g) dissolved in a mixture of 1N NaOH (16 mmol; 10 ml) [lacuna] in a three-necked flask fitted with a septum, through which is passed a flow of compressed air. A characteristic evolution of ethanethiol is observed.

The reaction medium is stirred for 24 hours at room temperature; a white precipitate forms. 1 ml of concentrated hydrochloric acid is then added. The precipitate is then filtered off on a sinter funnel and dried in the open air or in a desiccator.

The crude product is purified by recrystallization from 10 ml of a benzene/hexane mixture (1/1) to give 1.60 g of N-trifluoroacetyl-5-aminovaleric acid of formula (7) below:

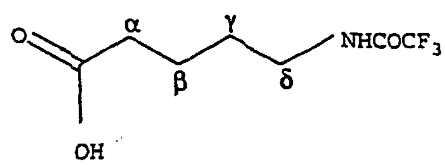

N-trifluoroacetyl-5-aminovaleric acid (compound 7)

**$^1$H NMR ($D_2O$):** 1.0 (m, 4H, $H_\beta$, $H_\gamma$); 2.2-2.3 (m, 2H, $H_\delta$); 3.2 (m, 2H, $H_\alpha$)

$^{13}$C NMR ($D_2O$): 26.5 ($C_\beta$); 30; 0 ($C_\gamma$); 34.1 ($C_\alpha$); 39.9 ($C_\delta$); 116.7 ($CF_3$, q, $^1J_{C-F}$=285.9 Hz); 157.6 ($\underline{C}OCF_3$, q, $^2J_{C-F}$=36.6 Hz ) ; 176.1 (COOH).

Example 4: Synthesis of N-trifluoroacetyl-6-amino-caproic acid: compound (8)

The process is performed in the same way as in Example 3, to give compound (8). The process is commenced using 1 equivalent of 6-aminocaproic acid (10 mmol; 1.31 g) dissolved in 1 equivalent of 1N NaOH (10 mmol; 10 ml), to which are added 1.6 equivalents of S-ethyl trifluorothioacetate (16 mmol; 2 ml). After purification, 1.70 g of N-trifluoroacetyl-6-amino-caproic acid (6b) (yield = 75%) of formula 8 below are recovered:

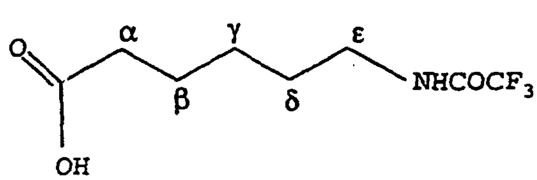

N-trifluoroacetyl-6-aminocaproic acid (compound 8)

**$^{1}H$ NMR ($D_2O$)**: 1.13-1.37 (m, 2H, $H_\gamma$); 1.45-1.54 (m, 4H, $H_\beta$, $H_\delta$); 2.21 (t, 2H, $^{3}J_{He\text{-}H\delta}$=7.7 Hz, $H_\varepsilon$); 3.16-3.25 (m, 2H, $H_\alpha$); 9.5 (s, 1H, NH).

**$^{13}C$ NMR (acetone):** 24.8 ($C_\beta$); 26.5 ($C_\gamma$); 30 ($C_\delta$); 34.1 ($C_\alpha$); 30.9 ($C_\varepsilon$); 116.7 ($CF_3$, q, $^{1}J_{C\text{-}F}$=285.9 Hz); 157.6 (<u>C</u>O$CF_3$, q, $^{2}J_{C\text{-}F}$=36.6 Hz) ; 176.1 (COOH).

<u>Example 5: Synthesis of N-trifluoroacetylornithine: compound (9)</u>

The protocol is identical to that described above: 1.6 equivalents of $EtSCOCF_3$ (16 mmol; 2 ml) are added to a solution of ornithine monohydrochloride (10 mmol; 1.68 g or 1.82 g, respectively) in a mixture of 1 N NaOH (10 mmol; 10 ml) [lacuna].

The crude product is purified by recrystallization from 10 ml of a water/ethanol mixture (1/1) to give 1.60 g of $N^{\delta}$-trifluoroacetylornithine of formula 9 below, in the form of a white powder, in a yield of 70%:

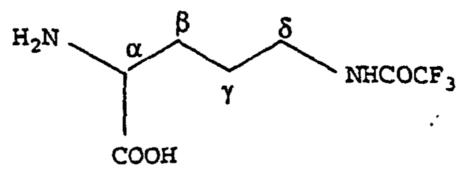

$N^{\delta}$-trifluoroacetylornithine (compound 9)

**$^{1}H$ NMR ($D_2O$):** 1.5-2 (m, 4H, $H_\beta$, $H_\gamma$); 3.2-3.4 (m, 2H, $H_\delta$), 4 (t, 1H, $^{3}J_{H\alpha\text{-}H\beta}$=6.1 Hz).

**$^{13}C$ NMR ($D_2O$):** 24.8 ($C_\gamma$) ; 28.2 ($C_\beta$); 39.6 ($C_\delta$); 54. 8 ($C_\alpha$); 116.3 ($CF_3$, q, $^{1}J_{C\text{-}F}$=285.2 Hz); 159 (<u>C</u>O$CF_3$, q, $^{2}J_{C\text{-}F}$=35 Hz); 174.9 (COOH).

<u>Example 6: Synthesis of N-trifluoroacetyllysine: compound (10)</u>

The protocol is identical to that described above: 1.6 equivalents of $EtSCOCF_3$ (16 mmol; 2 ml) are added to a solution of lysine monohydrochloride (10 mmol; 1.82 g) in a mixture of 1 N NaOH (10 mmol; 10 ml) [lacuna].

The crude product is purified by recrystallization from 10 ml of a water/ethanol mixture (1/1) to give 1.70 g of $N^{\varepsilon}$-trifluoroacetyllysine of formula 10 below, in the form of a white powder, in a yield of 70%.

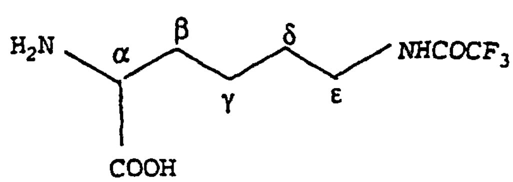

$N^{\varepsilon}$-trifluoroacetyllysine

(compound 10)

**$^{1}H$ NMR ($D_2O$):** 1.2-1.3 (m, 2H, $H_\delta$); 1.4-1.6 (m, 2H, $H_\gamma$), 1.7-1.8 (m, 2H, $H_\beta$); 3.2 (t, 2H, $^{3}J_{H\varepsilon\text{-}H\delta}$=6.5 Hz, $H_\varepsilon$); 3.6 (t, 1H, $^{3}J_{H\alpha\text{-}H\beta}$=6.2 Hz, $H_\alpha$).

**$^{13}C$ NMR ($D_2O$):** 25.9 ($C_\gamma$); 28.5 ($C_\delta$); 31.7 ($C_\beta$); 37.3 ($C_\varepsilon$); 46.8 ($C_\alpha$); 113.7 ($CF_3$, q, $^{1}J_{C\text{-}F}$=287.8 Hz); 155.2 (<u>C</u>O$CF_3$, q, $^{2}J_{C\text{-}F}$=37.14 Hz); 167.3 (COOH).

<u>Example 7: Synthesis of $N^{\varepsilon}$-trifluoroacetyldiaminopentane: compound (22)</u>

50 mmol of diaminopentane are dissolved in 100 ml of methanol. A solution of 50 mmol of ethyl trifluorothioacetate in 10 ml of methanol is added dropwise. The reaction mixture is stirred for 3 hours. After evaporating off the solvent, a yellow oil is obtained, which partially crystallizes in ice. This synthesis may be represented schematically in the following manner:

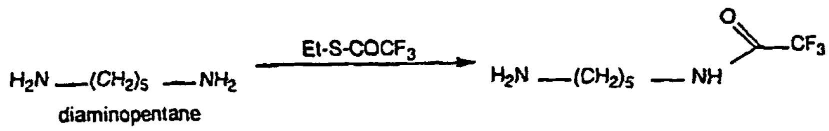


$N^{\varepsilon}$-trifluoroacetyldiaminopentane

(compound 22)

A purification is performed by flash chromatography on silica gel, with the eluent: $CH_2Cl_2$/MeOH (90/10).

**I.R. (KBr):** 3500-3400 $cm^{-1}$ (ν NH amide and amine, broad band), 2867 $cm^{-1}$ (ν $CH_2$ of the alkyl chain), 1711 $cm^{-1}$ (ν CO of the amide), 1490 $cm^{-1}$ (ν CH of s $CH_2$).

**$^1$H NMR (DMSO $D_6$):** 3.13 [t(J=7.05 Hz); 2H; $CH_2\alpha$]; 2.46 [t(J-6.54 Hz); 2H; $CH_2\varepsilon$]; 1.43 [m(J=7 Hz); 2H; $CH_2\beta$]; 1.24 [m; 4H; $CH_2\delta$ and γ].

**$^{13}$C NMR (DMSO):** 156.1 [q; C=0 of the trifluoroacetamide]; 116 [q; $CF_3$]; 41.5, 39.2, 32.8, 28.2, 23.6 [s, 5 $CH_2$].

Example 8: Synthesis of $N^{\alpha}$-Boc-diaminobutane: compound (27)

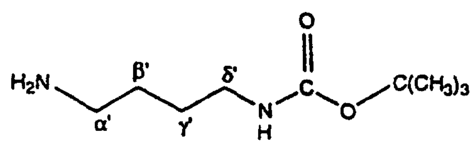

compound (27)

A solution of di-tert-butyl dicarbonate (4.9 g, 0.022 mol) in dioxane (60 ml) is added over a period of 2.5 hours to a solution of 1,4-butanediamine (15.51 g, 0.176 mol) in dioxane (60 ml). The mixture is stirred for 22 hours and the solvent is evaporated off on a rotavapour. Water (50 ml) is added to the residue and the insoluble disubstituted product is recovered by filtration. The filtrate is dried with anhydrous magnesium sulphate. Next, it is extracted with methylene chloride (3 × 50 ml). After evaporating off the solvent, 3.4 g of a colourless oil (compound 27) (81%) are obtained, and gradually solidified to give a white solid (m.p. = 112°C, Lit*.m.p. = 110-112°C).

*A.P. Krapcho, C.S. Kuell, Mono-protected diamines, N-tert-butoxycarbonyl-α-ω-alkanediamines From α, ω-Alkanediamines, Synthetic Communications, 1990, 20, 2559-2564.

**$^1$H NMR ($CDCL_3$):** δ(ppm)=1.28 (s, 2H, $NH_2$); 1.3-1.6 (m, 4H, $H_{\gamma}$, $H_{\beta'}$); 1.45 (s, 9H); 2.65 (t, 2H, $H_{\alpha'}$); 3.04 (q, 2H, $H_{\delta'}$); 4.7 (s1, 1H).

**$^{13}$C NMR** (50.2 MHz, $CDCl_3/CHCl_3$: 77 ppm/TMS): δ (ppm)=155.80 ($COOC(CH_3)_3$); 78.72 ($C(CH_3)_3$); 41.57 ($C_{\delta'}$); 40.16 ($C_{\alpha'}$); 30.62 ($C_{\gamma}$); 28.16 ($(CH_3)_3$); 27.24 ($C_{\beta'}$).

**C) EXAMPLES OF STEPS FOR ACTIVATING THE COOH FUNCTIONS OF THE COMPOUNDS OF FORMULA VI ACCORDING TO THE PROCESS OF THE INVENTION**

Example 9: Activation of the acid functions of compounds (7) and (8)

470 mg of compound (7) - or 500 mg of compound (8) - (2.20 mmol) are dissolved in 20 ml of ethyl acetate in a 50 ml round-bottomed flask. 1 equivalent of N-hydroxysuccinimide (2.20 mmol; 253 mg) is added. 1 equivalent of dicyclohexylcarbodiimide (2.20 mmol, 454 mg) is added to this clear solution. The mixture is stirred for 24 hours at room temperature. A white precipitate of N,N'-dihexylurea appears rapidly, and is removed by filtration through a sinter funnel. The filtrate is recovered and evaporated. A pale yellow oil is obtained, which is taken up, if necessary, in a small amount of ethyl acetate, and the mixture is refiltered. This operation removes the remaining urea. The filtrate is evaporated to give a transparent oil which crystallizes at room temperature to give 682 mg of compound (11) - or 713 mg

of compound (12) - in the form of a pearlescent white solid (yield = 90%).

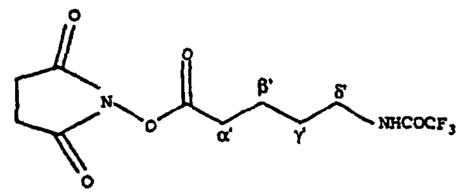

N'-succinimidyl N-trifluoroacetyl-5-aminovalerate

(compound 11)

**$^1$H-NMR ($CDCl_3$):** 1.25-1.9 (m, 4H, $H_\beta$, $H_\gamma$); 2.6 (t, 2H, $^3J_{H\delta'-H\gamma}$=6.3 Hz); 2.8 (s, 4H, $H_{succinimide}$); 3.2-3.4 (m, 2H, $H_{\alpha'}$); 7.4 ( s , 1H, NH).

**$^{13}$C NMR ($CDCl_3$):** 22.0 ($C_{\beta'}$); 30.7 ($C_\gamma$); 39,5 ($C_{\delta'}$); 60.8 ($C_{\alpha'}$); 119.1 ($CF_3$, q, $^1J_{C-F}$=287.6 Hz); 157.5 ($COCF_3$, q, $^2J_{C-F}$=36.78 Hz); 168.7 and 169.9 (2 $CO_{succinimide}$); 171.8 (CO-O).

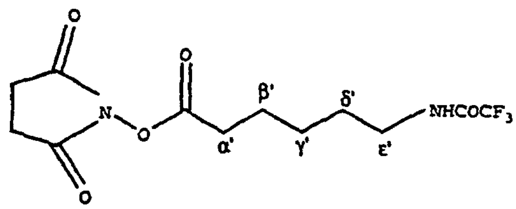

N'-succinimidyl N-trifluoroacetyl-6-aminocaproate

(compound 12)

**$^1$H-NMR ($CDCl_3$):** 1.2-2.0 (m, 6H, $H_\beta$, $H_\gamma$, $H_\delta$); 2.5 (t, 2H, $^3J_{H\varepsilon-H\delta}$=6.5 HZ); 2.8 (S, 4H, $H_{succinimide}$); 3.2-3.3 (m, 2H, $H_\alpha$); 7.5 (s, 1H, NH).

**$^{13}$C NMR ($CDCl_3$):** 21.4 ($C_\gamma$); 22.0 ($C_\beta$); 28.0 ($C_\delta$); 39.6 ($C_\varepsilon$); 60.8 ($C_\alpha$); 119.0 ($CF_3$, q, $^1J_{C-F}$=289.3 Hz); 157.5 ($\underline{C}OCF_3$, q, $^2J_{C-F}$=36.7 Hz ) ; 168.6 and 168.8 (2 $CO_{succinimide}$); 171.7 (CO-O).

<u>Example 10: Activation of hippuric acid with N-hydroxysuccinimide: compound (23)</u>

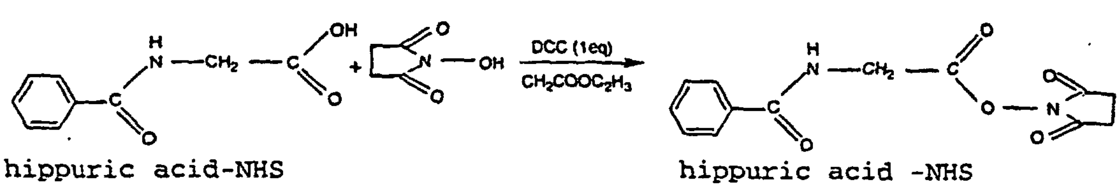


hippuric acid-NHS

hippuric acid -NHS

(compound (23))

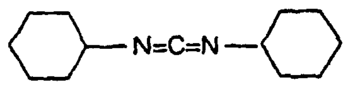

*DCC dicyclocarbodiimide*

17.3 mmol (2 g) of N-hydroxysuccinimide are dissolved in 60 ml of ethyl acetate. 17.3 mmol (3.1 g) of hippuric acid are added. A solution of 17.3 mmol (3.57 g) of dicyclohexylcarbodiimide in 15 ml of ethyl acetate is then added. A voluminous white precipitate forms. The reaction mixture is stirred for 15 hours. The solution is filtered and a white solid is recovered.

**Purification:**

Cold fractional recrystallization from ethyl acetate.
**m.p.** = 149-150°C.
**I.R. (KBr):** 3357 $cm^{-1}$ (strong band; v N-H of the amide), 1813, 1785, 1740 $cm^{-1}$ (v C=0 of the 4 carbonyl functions) 1640, 1579 $cm^{-1}$ (v of the aromatic rings).
**$^1H$ NMR (DMSO $D_6$):** 9.2 [t; 1H; NH amide]; 7.9 [d; 2H; aromatic $H_{ortho}$ ]; 7.5 [m; 3H; $H_{meta}$ and $H_{para}$]; 4.45 [d; 2H; aliphatic $CH_2$ ]; 2.8 [s; 4H; 2 cyclic $CH_2$ ].

Example 11 a): Coupling of hippuric acid and lysine to form compound (24)

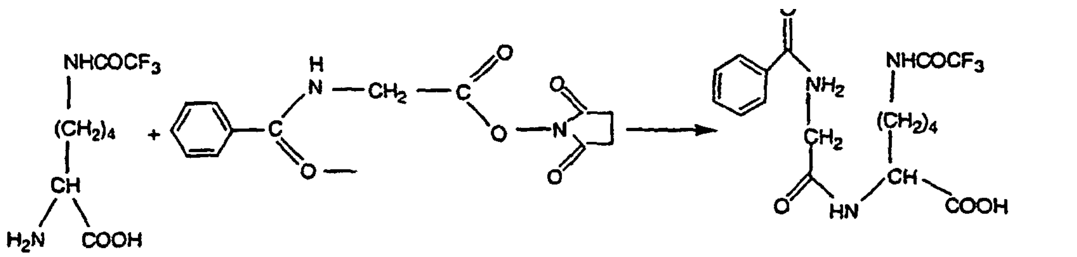

lysine compound (23) $N^{\varepsilon}$ trifluoroacetyl-lysine $N^{\alpha}$_hippurate (compound (24))

1.5 mmol (0.5 g) of compound 23 are dissolved in 20 ml of tetrahydrofuran. 1.5 mmol (0.4 g) of $N^{\varepsilon}$-trifluoroacetyllysine and 1.5 mmol (0.2 ml) of triethylamine are then added and, after stirring for 20 hours, the mixture is clear. The THF is evaporated off and the oil obtained is taken up in ethyl acetate and washed with water. The aqueous phase is acidified with a few drops of concentrated acetic acid and then extracted with dichloromethane. A white solid precipitates in the organic phase (product).
**$^1H$ NMR (DMSO $D_6$):** 9.4 [t; 1H, NH of the hippuric acid]; 8.7 [t; 1H; NH trifluoroacetamide]; 8.2 [d; 1H; amide NH of the coupling]; 7.8 [d; 2H, $H_{ortho}$]; 7.5 [m; 3H; $H_{para}$ and 2 $H_{meta}$]; 3.9 [t; 2H; $CH_2$ of the hippuric acid]; 3.1 [m; 2H; $CH_2\varepsilon$]; 1.1-1.8 [3 m; 6H; 3 $CH_2$ of the aliphatic chain].

Example 11 b): Activation of $N^{\varepsilon}$-trifluoroacetyllysine $N^{\alpha}$-hippurate: (compound 24) with N-hydroxysuccinimide to form compound (25)

10 mmol (2 g) of N-hydroxysuccinimide are dissolved in 60 ml of ethyl acetate. 10 mmol of $N^{\varepsilon}$-trifluoroacetyllysine $N^{\alpha}$-hippurate are added. A solution of 10 mmol of dicyclohexylcarbodiimide in 20 ml of ethyl acetate is added. The reaction mixture is stirred for 24 hours. The solution is filtered and a white solid is recovered.
**$^1H$ NMR (DMSO $D_6$):** 9.4 [t; 1H, NH of the hippuric acid]; 8.7 [t; 1H; NH trifluoroacetamide]; 8.2 [d; 1H; amide

NH of the coupling]; 7.8 [d; 2H, $H_{ortho}$]; 7.5 [m; 3H; $H_{para}$ and 2 $H_{meta}$]; 3.9 [t; 2H; $CH_2$ of the hippuric acid]; 3.1 [m; 2H; $CH_2\varepsilon$]; 2.8 [s; 4H; 2 $CH_2$]; 1.1-1.8 [3 m; 6H; 3 $CH_2$ of the aliphatic chain].

**Purification:**

Cold fractional recrystallization from ethyl acetate.

**D) EXAMPLES OF STEPS FOR LINKING A COMPOUND OF FORMULA V AND A COMPOUND OF FORMULA VI ACTIVATED VIA ITS CARBOXYL FUNCTION ACCORDING TO THE PROCESS OF THE INVENTION**

Example 12: Synthesis of $N^\delta$,$N^\delta$-bis(trifluoroacetyl)-N-(5-aminopentanoyl)ornithine: compound (13)

1 equivalent of compound (9) or [lacuna] (2 mmol; i.e. 456 mg) is dissolved in 10 ml of dimethylformamide in a 25 ml round-bottomed flask. A suspension is obtained, to which is added 1 ml of triethylamine and 1.5 equivalents of compound (11) (3 mmol; 639 mg or 681 mg, respectively). The reaction mixture is stirred for 24 hours at room temperature to give a clear solution.

The mixture is then made alkaline by successive additions of a sodium carbonate solution until a pH = 8 is obtained. Next, the mixture is extracted with twice 15 ml of ethyl acetate. The aqueous phase is recovered and hydrolysed with a few ml of concentrated hydrochloric acid until a pH = 2 is obtained. The resulting mixture is extracted with three times 15 ml of ethyl acetate. The organic phases are then washed with water to remove the remaining DMF, and then with brine and are dried over $Na_2SO_4$. The solvent is then evaporated off to give a clear oil which crystallizes at room temperature. The yield is 70%.

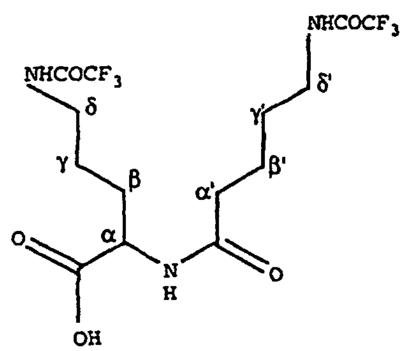

$N^\delta$,N-bis(trifluoroacetyl)-N-(5-aminopentanoyl)ornithine

(compound 13)

**$^1$H NMR (acetone):** 1.5-2.2 (2m, 8H, $H_\beta H_{\beta'}$, $H_\gamma$,$H_{\gamma}$); 2.3 (m, 2H, $H_{\alpha'}$); 3.4 (t, 4H, $H_\delta H_{\delta'}$, $^3J_{H\delta\text{-}H\gamma}$=6 Hz, $^3J_{H\delta'\text{-}H\gamma'}$= 6 Hz); 4.5 (m, 1H, $H_\alpha$); 7.6 (d, 1H, $^3J_{NH\text{-}H\alpha}$=7.6 Hz); 8.6 (s 2H, 2NHCOC$F_3$).

**$^{13}$C NMR (DMSO $d_6$):** 23.3 to 29.2 ($C_\beta C_{\beta'} C_\gamma C_{\gamma'}$); 36.6 ($C_{\alpha'}$); 41.1 and 41.9 ($C_\delta C_{\delta'}$); 52.3 ($C_\alpha$); 116.8 ($CF_3$, q, $^1J_{C\text{-}F}$=288.3 Hz); 157.0 ($\underline{C}OCF_3$, q, $^2J_{C\text{-}F}$=36.7 Hz); 172.9 (CONH); 174.5 (COOH).

Example 13: Synthesis of $N^\delta$,N-bis(trifluoroacetyl)-N-(5-aminohexanoyl)ornithine: compound (14)

The process is performed in the same manner as in Example 12 with 1 equivalent of compound (9) (2 mmol; i.e. 456 mg). Compound (11) is replaced with 1.5 equivalents of compound (12) (3 mmol; i.e. 681 mg). The yield is 70%.

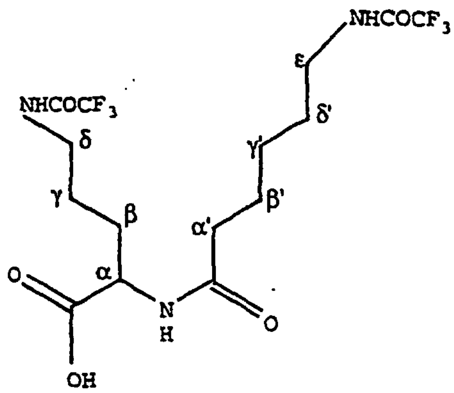

$N^{\delta},N^{\varepsilon'}$-bis(trifluoroacetyl)-N-(6-aminohexanoyl)ornithine

(compound 14)

**$^{1}$H NMR (DMSO $d_6$):** 1.1-1.8 (m, 10H, $H_{\beta}H_{\beta'}$, $H_{\gamma}H_{\gamma}H_{\delta'}$); 2.1 (t, 2H, $^{3}J_{H\alpha'-H\beta'}$=7.2 Hz, $H_{\alpha'}$); 3.2 (m, 4H, $H_{\varepsilon'}H_{\delta}$); 4.1 (m, 1H, $H_{\alpha}$); 8.1 (d, 1H, $^{3}J_{NH-H\alpha}$=7.7 Hz, NH); 9.4 (s, 2H, 2NHCOCF$_3$).
**$^{13}$C NMR (DMSO $d_6$):** 27.4 to 30.9 ($C_{\beta}C_{\beta'}C_{\gamma}C_{\gamma}C_{\delta'}$); 37 ($C_{\alpha'}$); 41 ($C_{\varepsilon'}C_{\delta}$); 54 ($C_{\alpha}$); 120 (2 CF$_3$, $^{1}J_{C-F}$=288.2 Hz); 158.6 (2 COCF$_3$, q, $^{2}J_{C-F}$=39.0 Hz); 174.9 (CONH); 176.2 (COOH).

Example 14: Synthesis of $N^{\delta}$,$N^{\delta}$-bis(trifluoroacetyl)-N-(5-aminopentanoyl)lysine: compound (15)

1 equivalent of compound (10) (2 mmol; i.e. 484 mg) is dissolved in 10 ml of dimethylformamide in a 25 ml round-bottomed flask. A suspension is obtained, to which is added 1 ml de triethylamine and 1.5 equivalents of compound (11) (3 mmol; i.e. 639 mg or 681 mg). The reaction mixture is stirred for 24 hours at room temperature to give a clear solution.
The mixture is then made alkaline by successive additions of a sodium carbonate solution until a pH = 8 is obtained. Next, the mixture is extracted with twice 15 ml of ethyl acetate. The aqueous phase is recovered and hydrolysed with a few ml of concentrated hydrochloric acid until a pH = 2 is obtained. The resulting mixture is extracted with three times 15 ml of ethyl acetate. The organic phases are then washed with water to remove the remaining DMF, and then with brine and are dried over $Na_2SO_4$. The solvent is then evaporated off to give a clear oil which crystallizes at room temperature. The yield is 70%.

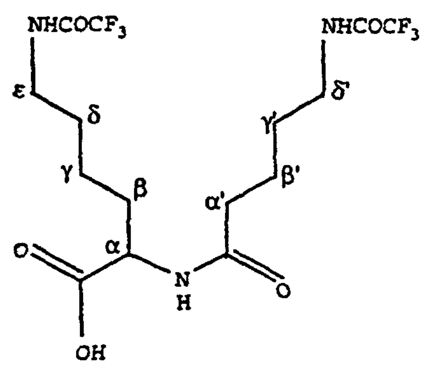

$N^{\varepsilon},N^{\delta'}$-bis(trifluoroacetyl)-N-(5-aminopentanoyl)lysine

(compound 15)

**$^{1}$H NMR (200 MHz, acetone):** 1.1-1.8 (m, 5H, $H_{\beta}H_{\beta'}$, $H_{\gamma}H_{\gamma}$); 2.2 (t, 2H, $^{3}J_{H\alpha'-H\beta'}$=6.5 Hz, $H_{\alpha'}$); 3.2 (m, 4H, $H_{\varepsilon'}H_{\delta'}$); 4 . 3 (m, 1H, $H_{\alpha}$); 7.5 (d, 1H, $^{3}J_{NH-H\alpha}$=7.8 Hz, NH); 8.4 (s, 2H, 2NHCOCF$_3$).
**$^{13}$C NMR (acetone):** 23 to 31 ($C_{\beta}C_{\beta'}C_{\gamma}C_{\gamma}$ and $C_{\delta}$); 39 ($C_{\alpha'}$); 49 ($C_{\varepsilon}C_{\delta'}$); 52 ($C_{\alpha}$); 117 (2 CF$_3$, $^{1}J_{C-F}$=287.4 Hz) ;

156 (2 $\underline{C}OCF_3$, q, $^2J_{C-F}$=35.3 Hz); 173.5 (CONH); 173.7 (COOH).

Example 15: Synthesis of $N^\delta$,$N^\delta$-bis(trifluoroacetyl)-N-(5-aminohexanoyl)lysine: compound (16)

The process is performed in the same manner as in Example 14, with 1 equivalent of compound (10) (2 mmol; i.e. 456 mg or 484 mg). Compound (11) is replaced with 1.5 equivalents of compound (12) obtained in Example 9 above (3 mmol; i.e. 681 mg). The yield is 70%.

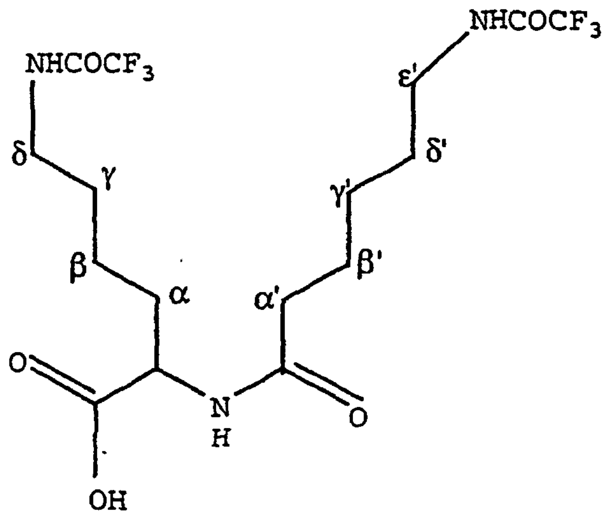

$N^\varepsilon$,$N^{\varepsilon'}$-bis(trifluoroacetyl)-N-(6-aminohexanoyl)lysine

(compound 16)

**$^1$H NMR (DMSO $d_6$):** 1.2-1.7 (3m, 12H, $H_\beta H_{\beta'} H_\gamma H_{\gamma} H_\delta H_{\delta'}$); 2.1 (t, 2H, $^3J_{H\alpha'-H\beta'}$=7.3 Hz, $H_{\alpha'}$); 3.1-3.25 (m, 4H, $H_{\varepsilon'} H_{\varepsilon'}$); 4.0-4.2 (m, 1H, $H_\alpha$); 8.1 (d, 1H, $^3J_{NH-H\alpha}$=7.7 Hz, NH); 9.5 (s, 2H, $2NHCOCF_3$); 12.3 (s, 1H, COOH).

**$^{13}$C NMR (DMSO $d_6$):** 23.5-34.3 ($C_\beta C_{\beta'} C_\gamma C_\gamma C_\delta C_{\delta'}$); 36.6 ($C_{\alpha'}$); 116.8 (2 $CF_3$, q, $^1J_{C-F}$=287.8 Hz); 156.8 (2 $\underline{C}OCF_3$, q, $^2J_{C-F}$=35.7 Hz); 173.1 (CONH); 174.6 (COOH).

Example 17: Coupling of $N^\varepsilon$-trifluoroacetyllysine $N^\alpha$-hippurate NHS (24) to $N^\varepsilon$-trifluoroacetyldiaminopentane (22) to form compound (26)

1.5 mmol of $N^\varepsilon$-trifluoroacetyllysine $N^\alpha$-hippurate NHS (24) are dissolved in 50 ml of tetrahydrofuran. 1.5 mmol of $N^\varepsilon$-trifluoroacetyldiaminopentane (22) and 1.5 mmol (0.2 ml) of triethylamine are then added and, after stirring for 20 hours, the mixture is clear. The THF is evaporated off and the oil obtained is taken up in ethyl acetate and washed with water. The aqueous phase is acidified with a few drops of concentrated acetic acid and then extracted with dichloromethane. A white solid precipitates in the organic phase.

This reaction may be represented schematically in the following manner:

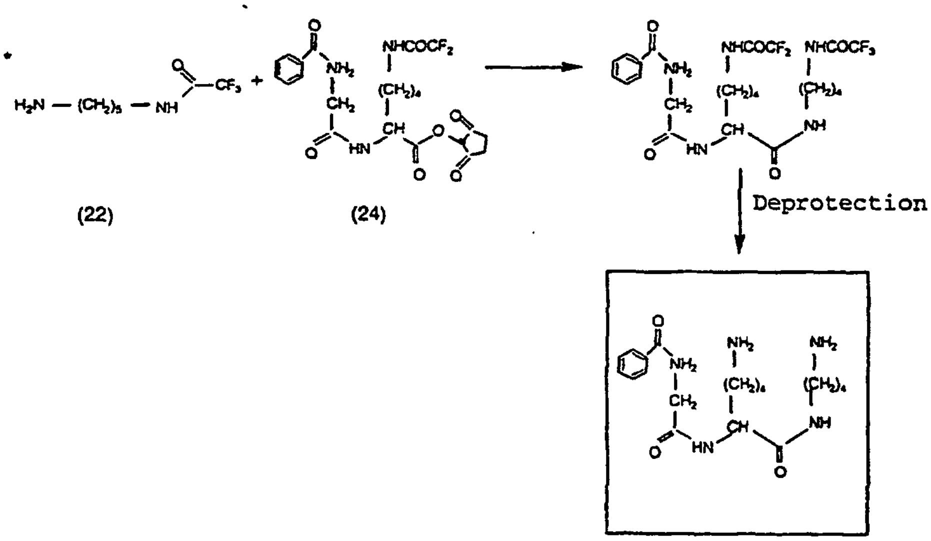


(22) (24)

(compound 26)

**$^1$H NMR (DMSO $D_6$):** 9.4 [t; 1H, NH of the hippuric acid]; 8.7 [t; 1H; NH trifluoroacetamide]; 8.2 [d; 1H; amide NH of the coupling]; 7.8 [d; 2H, $H_{ortho}$]; 7.5 [m; 3H; $H_{para}$ and 2 $H_{meta}$]; 3.9 [t; 2H; $CH_2$ of the hippuric acid]; 3.1-3.2 [m; 4H]; 2.4-2.5 [t; 2]; 1.1-1.8 [m; 12H; 6 $CH_2$ of the aliphatic chains].

Example 18: Synthesis of $N^{\varepsilon}$,$N^{\delta'}$-bis(tert-butoxycarbonyl)-($\alpha'$,$\delta'$-diaminobutyl)-$N^{\alpha}$(carboxybenzyloxy)-D-lysine: compound (28)

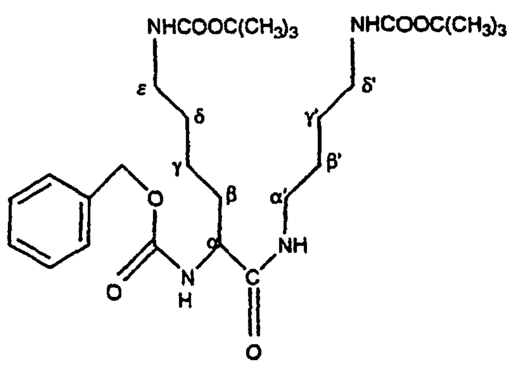

(compound 28)

0.200 g ($1.067\times10^{-3}$ mol) of N-tert-butoxycarbonyl-1,4-diaminobutane (compound (27)), 0.405 g ($1.067\times10^{-3}$ mol) of $N^{\alpha}$-CBz-$N^{\varepsilon}$-tBoc-D-lysine, 0.220 g ($1.067\times10^{-3}$ mol) of 1,3-dicyclohexylcarbodiimide and 0.144 g ($1.067\times10^{-3}$ mol) of 1H-hydroxybenzotriazole are dissolved in 30 ml of dry dichloromethane in a 50 ml round-bottomed flask. A white precipitate of N,N'-dicyclohexylurea appears during the reaction. The reaction mixture is stirred for 20 hours at room temperature. The filtrate is evaporated to give a white solid, which is dissolved in $H_2O$ (20 ml) and treated with a saturated $NaHCO_3$ solution (20 ml). The aqueous phase is extracted with dichloromethane (40 ml) and washed with water (10 ml). The organic phase is dried over $MgSO_4$ and evaporated to give a solid compound. The product is purified by flash chromatography (using various eluents: $CH_2Cl_2$ alone (100 ml) followed by EtOAc (200 ml). The pure fractions lead to 0.302 g, 52%, of solid product (compound (28)), m.p. = 60-62°C.

* **$^1$H NMR** (200.13 MHz, $CDCl_3/CHCl_3$): 7.24 ppm/TMS): δ(ppm)=7.3 (m, 5H, Ph-H); 6.4 (s, 1H, NHCOO); 5.5 (s1, 1H, NHCO); 5.07 (m, 1H, Ph-$CH_2O$); 4.07 (m, 1H, $H_\alpha$); 3.24 (m, 1H, $H_{\alpha'}$); 3.06 (m, $H_\varepsilon$, $H_{\delta'}$); 1.40 (m, 9H, $(CH_3)_3$); 1.87-1.23 (m, 5H, $H_\beta$, $H_{\beta'}$, $H_\delta$, $H_\gamma$, $H_{\gamma'}$).
* **$^{13}$C NMR** (50.2 MHz, $CDCl_3/CHCl_3$: 77 ppm/TMS): δ(ppm)=171.57 (NHCO) : 156.09 & 155.99 (2xCOOC$(CH_3)_3$); 136.01 (aromatic C); 128.32 (aromatic $CH_2$); 128.00 (aromatic CH) ; 127.90 (aromatic $CH_2$); 79.13 (2xC$(CH_3)_3$); 66.81 (Ph-$CH_2$); 54.72 ($C_\alpha$); 39.94 ($C_{\delta'}$); 39.75 ($C_\varepsilon$); 31.90 ($C_\delta$); 29.41 ($C_{\gamma'}$); 28.19 ($(CH_3)_3$); 27.36 ($C_\gamma$); 26,15 ($C_{\beta'}$); 22.26 ($C_\beta$).

* **Infrared**

1689 $cm^{-1}$ : ν(NHCOO); 1652 $cm^{-1}$ : ν(NHCO).

* **Mass spectrum**

The molar mass is 550.

The peak 573 corresponds to (M'+Na).

Synthesis of $N^\varepsilon$,$N^{\delta'}$-bis(tert-butoxycarbonyl)-(α',δ'-diaminobutyl)-D-lysine: compound (29)

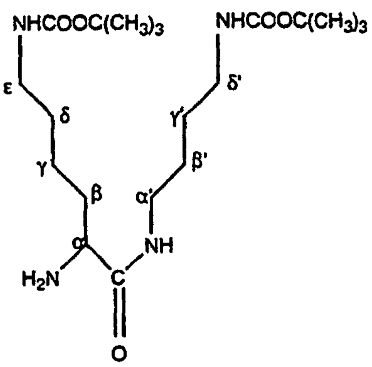

(compound 29)

0.300 g ($5.49\times10^{-4}$ mol) of $N^\varepsilon$,$N^{\delta'}$-bis(tert-butoxycarbonyl)-(α',δ'-diaminobutyl)-$N^\alpha$(carboxybenzyloxy)-D-lysine (compound (28)) is dissolved in 20 ml of methanol in a 50 ml round-bottomed flask, and 40 mg of Pd/C (10%) are added. The reaction mixture is stirred for 60 hours; the Pd is removed by filtration using Celite. The solvent is removed under vacuum to give a colourless liquid. This liquid, washed with hexane and dried, gives 0.205 g (90%) of compound (29) (liquid).

* **$^1$H NMR** (200.13 MHz, $CDCl_3/CHCl_3$: 7.24 ppm/TMS): δ(ppm)=4.76 (m, 1H, $H_\alpha$); 3.18 (m, 1H, $H_\alpha$); 3.04 (m, 4H, $H_\varepsilon$, $H_{\delta'}$); 1.37 (m, 9H, $(CH_3)_3$); 1.68-1.37 (m, 5H, $H_\beta$, $H_{\beta'}$, $H_\delta$, $H_{\gamma'}$, $H_\gamma$).
* **$^{13}$C NMR** (50.2 MHz, $CDCl_3/CHCl_3$: 77 ppm/TMS): δ(ppm)=174.18 (NHCO): 155.89 (2xCOOC$(CH_3)_3$); 78.85 (2xC $(CH_3)_3$); 54.62 ($C_\alpha$); 39.91 ($C_{\delta'}$); 38.55 ($C_\varepsilon$); 34.03 ($C_\alpha$); 32.57 ($C_\delta$); 29.60 ($C_{\gamma'}$); 28.19 ($(CH_3)_3$); 27.27 ($C_\gamma$); 26.60 ($C_{\beta'}$); 22.54 ($C_\beta$).

**E) EXAMPLE OF A PROCESS FOR MANUFACTURING A BIS-DITHIOCARBAMATE COMPOUND ACCORDING TO THE PRESENT INVENTION IN WHICH A RADICAL R IS ATTACHED TO AN INTERMEDIATE PRODUCT OF THIS COMPOUND**

Example 19: Synthesis of methyl $N^{\delta}$,$N^{\delta}$-bis(trifluoroacetyl)-N-(5-aminohexanoyl)lysinate: compound (17)

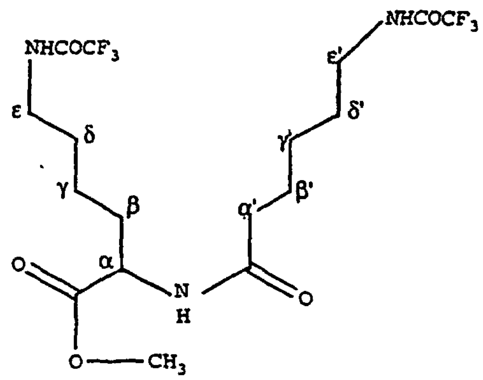

methyl $N^{\varepsilon}$,$N^{\varepsilon'}$-bis(trifluoroacetyl)-N-(6-amino-hexanoyl)lysinate

(compound 17)

100 mg (0.2 mmol) of compound (16) are dissolved in 10 ml of absolute methanol. 2 equivalents of TMCS (0.4 mmol; 56 µl) are added slowly to this suspension and the mixture is stirred for 24 hours at room temperature. The solvent is evaporated off to give a yellow oil, which is dissolved in 20 ml of ethyl acetate. The organic phase is washed with 20 ml of a sodium carbonate solution and then with 20 ml of brine and dried over $Na_2SO_4$. The solvent is evaporated off to give 90 mg of methyl $N^{\varepsilon}$,$N^{\varepsilon'}$-bis (trifluoroacetyl)-N-(6-aminohexanoyl)lysinate (compound (17)) in a yield of 90%.

**$^1$H NMR ($CDCl_3$):** 1.25-2.0 (m, 6H, $H_{\beta}$, $H_{\beta'}$, $H_{\gamma}$, $H_{\gamma}$, $H_{\delta}$, $H_{\delta'}$); 2.6 (m, 2H, $H_{\alpha'}$); 3.4 (m, 4H, $H_{\varepsilon}$, $H_{\varepsilon'}$); 3.7 (s, 3H, $CH_3$); 4.6 (m, 1H, $H_{\alpha}$); 6.5 (d, 1H, $^3J_{NH-H\alpha}$=7.1 Hz); 7.4 (s, 2H, 2 $NHCOCF_3$).

**IR (cm$^{-1}$):** 3104, 3238, 3417, 3480 (<u>NH</u>$COCF_3$ and -NH-), 1743 (C=0 ester).

Example 20: Synthesis of 2β-[$N^{\delta}$,$N^{\delta}$-bis(trifluoroacetyl)-N-(5-aminopentanoyl) ornithyloxymethyl]-3β-(4'-tolyl)-tropane: compound. (18)

0.8 equivalent of compound (13) obtained in Example 12 above (0.65 mmol, i.e. 275 mg) is added to a solution of 2β-hydroxymethyl-3β-(4'-tolyl)tropane (200 mg; 0.8 mmol) stirred at room temperature and under an inert atmosphere, for example nitrogen, in 20 ml of dichloromethane.

0.8 equivalent of DMAP (0.65 mmol; 80 mg) and 0.8 equivalent of EDCI (0.65 mmol, 125 mg) are then added. After stirring for 15 hours, the reaction medium is washed with an $NaHCO_3$ solution (20 ml) and then with 1N hydrochloric acid solution (20 ml) and finally with brine (20 ml), and the organic phases are dried over $Na_2SO_4$ and then filtered. The solvent is evaporated off and a yellow oil is recovered in a yield of 50%.

**$^1$H NMR ($CDCl_3$):** 1.3-1.5 (m, 9H, $H_2$, $H_{\beta}$, $H_{\beta'}$, $H_{\gamma}$, $H_{\gamma'}$); 1.5-1.9 (m, 3H, $H_{4\alpha}$, $H_{6\alpha}$, $H_{7\alpha}$); 2.0-2.1 (m, 4H, $H_{6\beta}$, $H_{7\beta}$, $H_{\alpha'}$); 2.2 (s, 3H, pH-$CH_3$); 2.3 (s, 3H, N-$CH_3$); 3.0-3.6 (m, 8H, $H_1$, $H_3$, $H_5$, $H_{4\beta}$, $H_{\delta}$, $H_{\delta'}$); 3.8-4.0 (m, 1H, $H_{\alpha}$); 4.3-4.5 (m, 2H, $H_8$); 6.25 (m, 1H, NH); 7.1-7.2 (m, 4H, aromatic H); 7.5 (m, 2H, 2 $NHCOCF_3$).

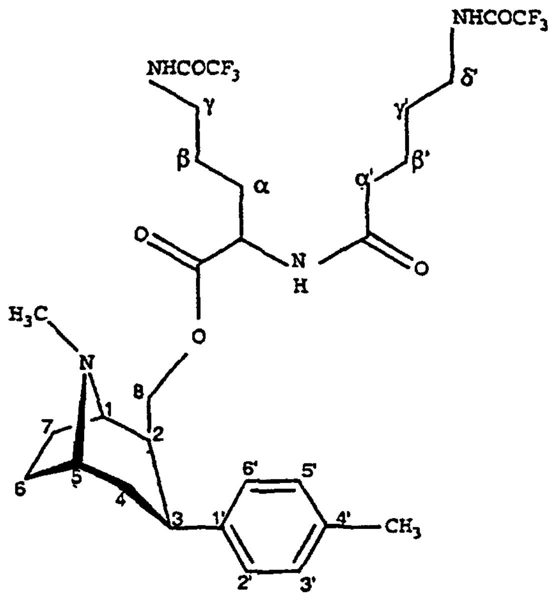

2β-[N$^{\delta}$,N$^{\delta'}$-bis(trifluoroacetyl)-N-(5-aminopentanoyl)-ornithyloxymethyl]-3β-(4'-tolyl)tropane (compound 18)

**$^{13}$C NMR ($CDCl_3$):** 21.2 (Ph-$CH_3$); 22.6 and 25.0 ($C_{\beta}$ and $C_{\beta'}$); 25.2 ($C_6$); 26.3 ($C_7$); 28.5 and 28.6 ($C_{\gamma}$ and $C_{\gamma'}$); 34.2 ($C_{\alpha'}$); 34.7 ($C_3$); 36.2 ($C_4$); 39.8 and 40.0 ($C_{\delta}$ et $C_{\delta'}$); 42.3 ($NCH_3$); 45.7 ($C_2$); 52.1 ($C_{\alpha}$); 62.3 ($C_5$); 64.1 ($C_8$); 65.6 ($C_1$); 116.4 ($CF_3$, q, $^1J_{C-F}$=287.4 Hz); 127.8 ($C_{2'}$ and $C_{6'}$); 129.4 ($C_{3'}$, $C_{5'}$); 136.2 ($C_{1'}$); 139 ($C_{4'}$); 157.6 (2 $COCF_3$, q, $^2J_{C-F}$=29.3 Hz); 172.5 (CONH); 173.4 and 173.6 (CO-0)- resolved signal.

Example 21: Synthesis of 2β-[N$^{\varepsilon}$,N$^{\varepsilon}$-bis(trifluoroacetyl)-N-(5-aminohexanoyl) lysinyloxymethyl]-3β-(4'-tolyl)-tropane: compound (19)

The process is performed in the same manner as in Example 20, with a solution of 2β-hydroxymethyl-3β-(4'-tolyl) tropane (200 mg; 0.8 mmol). Compound (13) is replaced with 0.8 equivalent of compound (16) obtained in Example 15 above, (0.65 mmol; i.e. 284 mg). The solvent is evaporated off and a yellow oil is recovered in a yield of 50%.

**$^1$H NMR ($CDCl_3$)**: 1.1-1.4 (m, 13H, $H_2$, $H_{\beta}$, $H_{\beta'}$, $H_{\gamma}$, $H_{\gamma'}$, $H_{\delta}$, $H_{\delta'}$); 1.4-1.8 (m, 3H, $H_{4\alpha}$, $H_{6\alpha}$, $H_{7\alpha}$); 1.9-2.1 (m, 4H, $H_{6\beta}$, $H_{7\beta}$, $H_{\alpha'}$); 2.2 (s, 3H, ph-$CH_3$); 2.3 (s, 3H, N-$CH_3$); 3.0-3.4 (m, 8H, $H_1$, $H_3$, $H_5$, $H_{4\beta}$, $H_{\varepsilon}$, $H_{\varepsilon'}$); 3.6-3.85 (m, 2H, $H_8$); 6.2 (m, 1H, NH); 7.1 (m, 4H, aromatic H); 7.5 (m, 2H, 2 $NHCOCF_3$).

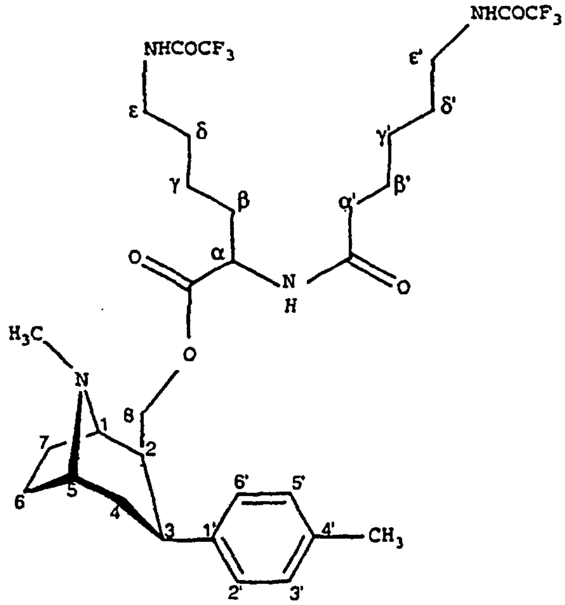

2β-[N$^{\varepsilon}$,N$^{\varepsilon'}$-bis(trifluoroacetyl)-N-(6-aminohexanoyl)lysinyloxymethyl]-3β-(4'-tolyl)tropane (compound 19)

Example 22 : Synthesis of 2β-[N$^{\delta}$, N$^{\varepsilon}$-bis(trifluoroacetyl)-N-(5-aminohexanoyl)ornithyloxymethyl]-3β-(4'-tolyl)-tropane: compound (20)

The process is performed in the same manner as in Example 20, with a solution of 2β-hydroxymethyl-3β-(4'-tolyl)tropane (200 mg; 0.8 mmol). Compound (13) is replaced with 0.8 equivalent of compound (14) obtained in Example 13 above (0.65 mmol; i.e. 284 mg). The solvent is evaporated off and a yellow oil is recovered in a yield of 50%.

**$^1$H NMR ($CDCl_3$):** 1.2-1.4 (m, 11H, $H_2$, $H_\beta$, $H_{\beta'}$, $H_\gamma$, $H_{\gamma'}$, $H_\delta$); 1.4-1.9 (m, 3H, $H_{4\alpha}$, $H_{6\alpha}$, $H_{7\alpha}$); 1.9-2.1 (m, 4H, $H_{6\beta}$, $H_{7\beta}$, $H_{\alpha'}$); 2.2 (s, 3H, ph-$CH_3$); 2.3 (s, 3H, N-$CH_3$); 3.0-3.5 (m, 8H, $H_1$, $H_3$, $H_5$, $H_{4\beta}$, $H_\delta$, $H_{\varepsilon'}$); 3.6-3.9 (m, 1H, $H_8$); 4.2-4.5 (m, 2H, $H_8$); 6.25 (m, 1H, NH) ; 7.0 (m, 4H, aromatic H) ; 7.4 (m, 2H, 2 $NHCOCF_3$).

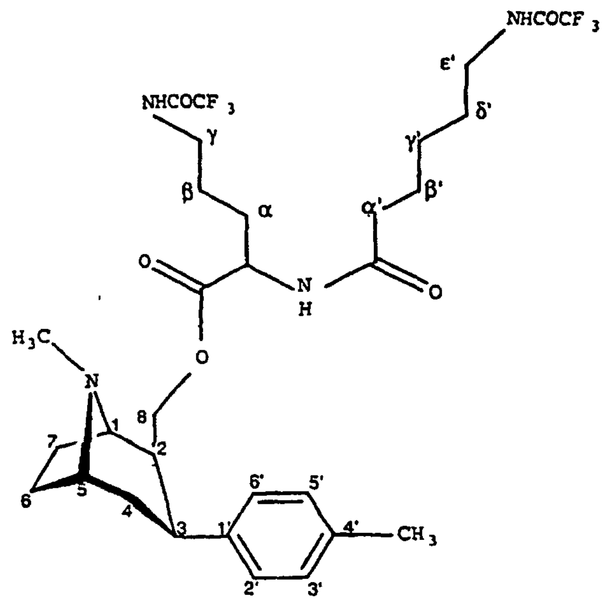

2β-[$N^{\delta}$,$N^{\varepsilon'}$-bis(trifluoroacetyl)-N-(6-aminohexanoyl)-ornithyloxymethyl]-3β-(4'-tolyl)tropane (compound 20)

Example 23: Synthesis of 2β-[$N^{\varepsilon}$,$N^{\delta}$-bis(trifluoroacetyl)-N-(5-aminopentanoyl)lysinyloxymethyl]-3β-(4'-tolyl)-tropane: compound (21)

The process is performed in the same manner as in Example 20 with a solution of 2β-hydroxymethyl-3β-(4'-tolyl) tropane (200 mg; 0.8 mmol). Compound (13) is replaced with 0.8 equivalent of compound (15) obtained in Example 14 above (0.65 mmol; i.e. 293 mg). The solvent is evaporated off and a yellow oil is recovered in a yield of 50%.

**$^1$H NMR ($CDCl_3$):** 1.1-1.4 (m, 11H, $H_2$, $H_\beta$, $H_{\beta'}$, $H_\gamma$, $H_\gamma$); 1.4-1.8 (m, 3H, $H_{4\alpha}$, $H_{6\alpha}$, $H_{7\alpha}$); 2.0-2.1 (m, 4H, $H_{6\beta}$, $H_{7\beta}$, $H_{\alpha'}$); 2.2 (s, 3H, ph-$CH_3$); 2.3 (s, 3H, N-$CH_3$); 2.7-3.9 (m, 1H, $H_\alpha$); 4.2-4.5 (m, 2H, $H_8$); 4.2-4.5 (m, 2H, $H_8$); 6.2 (m, 1H, NH) ; 7.0 (m, 4H, aromatic H) ; 7.4 (m, 2H, 2 $NHCOCF_3$).

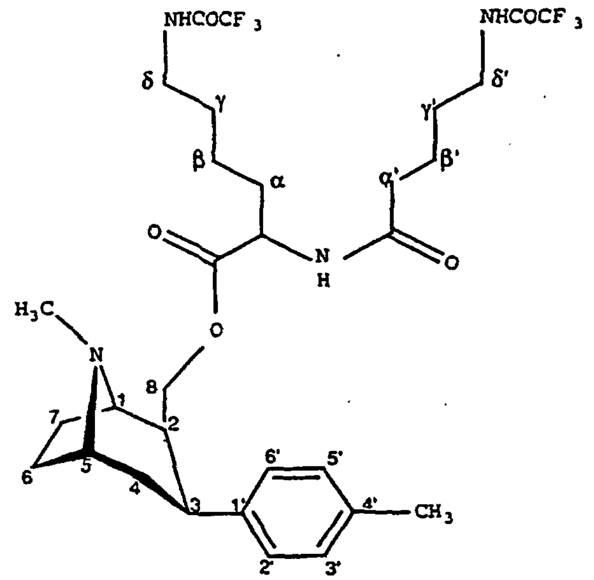

2β-[N$^{\varepsilon}$,N$^{\delta'}$-bis(trifluoroacetyl)-N-(6 aminopentanoyl)-lysinyloxymethyl]-3β-(4'-tolyl)tropane (compound 21)

Example 24 : 2β-[N$^{\varepsilon}$,N$^{\delta'}$-bis(tert-butoxycarbonyl)-(α',δ'-diaminobutyl)-D-lysine]-3β-(4'-tolyl)tropane: compound (30)

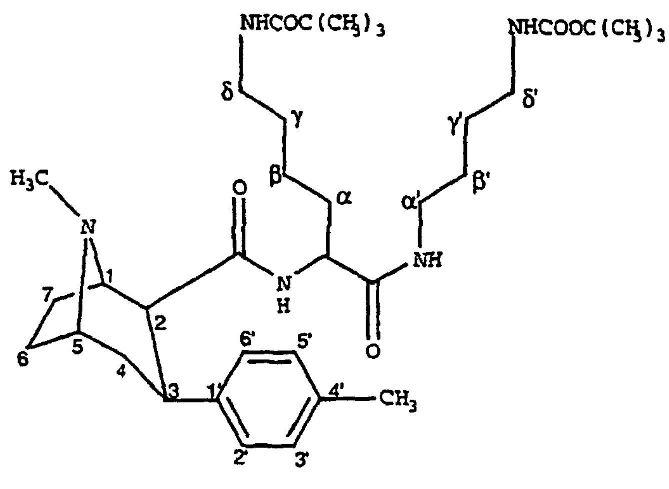

(compound (30))

0.173 g (4.15×10$^{-4}$ mol), of N$^{\varepsilon}$,N$^{\delta'}$-bis(tert-butoxycarbonyl)-(α',δ'-diaminobutyl)-D-lysine (compound 29), 0.085 g (4.15×10$^{-4}$ mol) of 1,3-dicyclohexylcarbodiimide, 0.056 g (4.15×10$^{-4}$ mol) of 1H-hydroxybenzotriazole and 0.123 g (4.15×10$^{-4}$ mol) of 2-β-carboxy-3β-tolyltropane are dissolved in 30 ml of dry dichloromethane in a 50 ml round-bottomed flask. A white precipitate of N,N'-dicyclohexylurea appears during the reaction. The reaction mixture is stirred

for 20 hours at room temperature. The filtrate, recovered and evaporated, gives a white solid which is dissolved in water $H_2O$ (10 ml) and treated with saturated $NaHCO_3$ solution (10 ml). The aqueous phase is extracted with dichloromethane (3×20 ml) and washed with water (10 ml); the organic phase obtained is dried over $MgSO_4$ and evaporated to give a liquid compound. The product is purified by flash chromatography (eluent: EtOAc (300 ml)). 0.09 g, 33%, of a colourless liquid product (compound 30) is obtained.

* **$^1$H NMR** (200.13 MHz, $CDCl_3/CHCl_3$: 7.24 ppm/TMS): δ(ppm)=8.6 (m, 2H, NHCO); 7.23-7.6 (m, 4H, Ph-H); 6.2 (m, 1H, NH) ; 4.23 (m, 2H, $H_8$); 2.9-3.2 (m, 3H, $H_\alpha$, $H_\varepsilon$, $H_{\delta'}$); 2.1 (s, 3H, N-$CH_3$); 2.3 (s, 3H, Ph-$CH_3$); 1.89 (m, 4H, $H6_\beta$, $H7_\beta$, $H_{\alpha'}$); 1.3-1.6 (m, 3H, $H4_\alpha$, $H6_\alpha$, $H7_\alpha$); 1.35 (m, 9H, $(CH_3)_3$); 1.03-1.21 (m, 11H, $H_2$, $H_\beta$, $H_{\beta'}$, $H_\delta$, $H_\gamma$, $H_\gamma$).
* **$^{13}$C NMR** (50.2 MHz, $CDCl_3/CHCl_3$: 77 ppm/TMS): δ(ppm)=169.6 (NHCO): 168.03 (NHCO); 156.73 (2xCOOC $(CH_3)_3$); 141.76 ($C_{4'}$); 128.42 ($C_{4'}$); 126.22 and 125.90 ($C_{3'}$, $C_{5'}$); 117.86 ($C_{3'}$); 79.43 (2xC$(CH_3)_3$); 75.79 (C4); 62.07 (C5); 54.03 ($C_\alpha$); 49.59 ($C_2$); 41.55 (NHCH$H_3)_3$); 40.40 ($_{c\delta}$'); 39.70 ($_{c\varepsilon}$); 34.11 (C4); 31.43 ($C_\varepsilon$); 29.99 ($C_\gamma$); 29.64 ((CH3)$_3$); 28.78 (C6); 26.52 ($C_{\beta'}$); 22.54 ($C_\beta$); 22.40 (Ph-$CH_3$).

* **Infrared**

1689 $cm^{-1}$: v(NHCOO); 1678 $cm^{-1}$: v(NHCO)

* **Mass spectrum**

The molar mass 4 is 657.
Peak 658 corresponds to (M+H).

**F) EXAMPLES OF STEPS FOR DEPROTECTING THE XH FUNCTIONS AND FOR REACTING THESE DEPROTECTED FUNCTIONS WITH $CS_2$ TO FORM A BIS-DITHIOCARBAMATE STRUCTURE ACCORDING TO THE PROCESS OF THE PRESENT INVENTION**

Example 25: Synthesis of $N^\varepsilon$,$N^{\delta'}$-bis-dithiocarbamate)-(α',δ-diaminobutyl)-$N^\alpha$(carboxybenzyloxy)-D-lysine: compound (31)

5 mg of compound (28) are dissolved in 500 µl of absolute methanol. 100 µl of trifluoroacetic acid are added. The mixture is stirred for 30 minutes. The mixture is evaporated under vacuum. When the reaction mixture is dry, 500 µl of methanol and 500 µl of piperidine are added. The mixture is left for a further 30 minutes. The reaction mixture is then evaporated to dryness under vacuum. 1 ml of methanol and 200 ml of carbon sulphide ($CS_2$) are then added. The reaction mixture is stirred at room temperature for 2 hours and then evaporated to dryness. Compound (31) is maintained dry at -18°C.

Example 26 : 2β-[$N^\varepsilon$,$N^{\delta'}$-bis(dithiocarbamate)-(α',δ'-diaminobutyl)-D-lysine]-3β-(4'-tolyl)tropane: compound (32)

The process is performed in the same manner as Example 25, but with 5 mg of compound (30). The reaction mixture is stirred at room temperature for 2 hours and then evaporated to dryness. Compound (32) obtained is maintained dry at -18°C.

Example 27: The deprotection and synthesis of the bis-dithiocarbamates (non-functionalized complexing agents) of products (13), (14), (15), (16) and (17) obtained in the preceding examples leads to the corresponding compounds (34), (35), (36), (37) et (38)

10 mmol of each peptide are dissolved in 5 ml of absolute methanol. 5 ml of a 0.1M solution of piperidine in methanol are added and the mixture is stirred for 1 hour. The mixture is evaporated under vacuum.

The dry residue is taken up in 5 ml of methanol and 3 ml of carbon sulphide are added. The mixture is stirred for 2 hours. The reaction mixture is evaporated to dryness and is stored at -18°C.

Example 28: Deprotection and synthesis of the bis-dithiocarbamates (substituted with a tropane derivative) of products (18), (19), (20) and (21) obtained in the preceding examples leads to compounds (44), (45), (46) and (47)

The process is performed in the same manner as in Example 27, but with the products (18), (19), (20) and (21). Compounds (44), (45), (46) and (47) are obtained.

## G) RADIOLABELLING OF COMPOUNDS ACCORDING TO THE INVENTION

Example 29: The radiolabelling of products (31), (34), (35), (36), (37) and (38) with TcN leads to compounds (33), (39), (40), (41), (42) and (43)

Synthesis of the TcN intermediate

100 µg of tin chloride, 5 mg of SDH (succinyl dihydrazide) and 5 mg of PDTA (1,2-propanediamino-N,N,N',N'-tetraacetic acid) were freeze-dried in a labelling flask. 3 ml of $TcO^-_4$ (60 mCi) are added to this lyophilizate. The mixture is left to act for 15 minutes.

Complexation:

2 mg of *bis*-dithiocarbamate in 1 ml of ethanol are added to 1 ml of TcN. The mixture is left to react for one hour. The reaction is analysed by HPLC (reverse-phase, methanol-water). Labelling yield >95%.

The radiolabelling of compound 38 showed that the radiolabelled molecule, isolated by HPLC and left at room temperature, was stable for more than 4 hours.

Example 29a: Radiolabelling of products (31), (34), (35), (36), (37) and (38) with copper-64

2 mg of bis-dithiocarbamate (products 31, 34, 35, 36, 37 or 38) in 0.5 ml of ethanol are added to 1 ml of 0.1 M pH 5.5 ammonium acetate buffer containing 2 mCi of $^{64}$Cu-acetate. The mixture is left to act for one hour. The reaction is analysed by HPLC (reverse-phase, methanol-water).

The labelling yield is greater than 95% for each of the products.

Example 30: Radiolabelling of products (32), (44), (46) and (47) with TcN leads to products (48), (49), (50), (51) and (52)

The process is performed in the same manner as in Example 29, but with products (32), (44), (45), (46) and (47). Products (48), (49), (50), (51) and (52) are obtained.

Example 30a: Radiolabelling of products (32), (44), (46) and (47) with copper-64 leads to products (48), (49), (50), (51) and (52)

The process is performed in the same manner as in Example 29a, but with products (32), (44), (45), (46) and (47).

The labelling yield is greater than 95% for each of the products.

## H) EXAMPLES OF THE USE OF THE COMPOUND OF THE PRESENT INVENTION

Example 31: Bioavailability of Lys-Lys bis-dithiocarbamate in rats

The molecule was radiolabelled as described in Example (29). The radiolabelled compound is then isolated by HPLC, evaporated and taken up in 0.9% saline medium. The bioavailability in rats gives the following results (see Table 1):

- time 30 minutes and 60 minutes: 3 animals per point,
- time 3 hours: 2 animals per point,
- time 24 hours: 1 animal per point.

The results are expressed as a percentage of dose injected per organ.

The rats' urine was collected 1 hour 30 minutes after injection and analysed by HPLC. The result of this analysis is 87% of unchanged complex.

Table 1

| Organs | Average 30 min | Average 1 h | Average 3 h | Average 24 h |
|---|---|---|---|---|
| blood | 0.67 | 0.34 | 0.20 | 0.11 |

Table 1 (continued)

| Organs | Average 30 min | Average 1 h | Average 3 h | Average 24 h |
|---|---|---|---|---|
| liver | 14.38 | 9.08 | 3.84 | 1.33 |
| kidneys | 4.55 | 4.18 | 3.87 | 2.65 |
| adrenals | 0.02 | 0.01 | 0.01 | 0.00 |
| spleen | 0.13 | 0.09 | 0.05 | 0.04 |
| lungs | 0.43 | 0.28 | 0.15 | 0.08 |
| heart | 0.21 | 0.10 | 0.06 | 0.03 |
| bladder | 0.47 | 0.32 | 0.05 | 0.01 |
| urine | 14.39 | 14.70 | 0.44 | 0.05 |
| stomach | 8.24 | 3.36 | 2.64 | 0.08 |
| intestine | 20.55 | 32.92 | 51.4 | 0.49 |
| caecum | 0.31 | 0.18 | 0.11 | 0.66 |
| colon | 0.46 | 0.23 | 0.19 | 0.87 |
| brain | 0.03 | 0.01 | 0.01 | 0.01 |

Example 32: Biological results of compounds (48) and products (50), (51) and (52)

The model chosen is the rat. Depending on the compounds, we performed one or two sacrifice times (30 minutes or 1 hour).

At the times chosen, the brains were removed and the areas of interest were isolated and counted. From these results, we deduced the following:

- the crossing of the blood-brain barrier by the compound under consideration,
- a striatum/cerebellum ratio.

Table II below collates the results of this experiment.

Table II

| TcN tropane-bis-dithiocarbamate approach | | | | |
|---|---|---|---|---|
| Product | Precursor | Biological results | | |
| | | Biodistribution | S/C | Kapp |
| 50 | 2β-[$N^{\varepsilon}$,$N^{\varepsilon}$-bis(dithiocarbamate)-N-(5-aminohexanoyl)lysinyloxymethyl]-3β-(4'-tolyl)tropane<br>n=4<br>m=3 | 30': cerebellum: 0.168%<br>striatum: 0.167%<br>crossing of blood-brain barrier: 0.483%<br><br>1 h: cerebellum: 0.077%<br>striatum: 0.075%<br>crossing of blood-brain barrier: 0.217% | 1.00<br><br><br><br>0.97 | |

Table II (continued)

| TcN tropane-bis-dithiocarbamate approach | | | | |
|---|---|---|---|---|
| Product | Precursor | Biological results | | |
| | | Biodistribution | S/C | Kapp |
| 51 | 2β-[$N^{\delta}$,$N^{\varepsilon}$-bis(dithiocarbamate)-N-(5-aminohexanoyl)ornithyloxymethyl]-3β-(4'-tolyl)tropane<br>n=3<br>m=4 | 30': cerebellum: 0.113%<br>striatum: 0.126%<br>crossing of blood-brain barrier: 0.344%<br><br>1 h: cerebellum: 0.069%<br>striatum: 0.071 %<br>crossing of blood-brain barrier: 0.200% | 1.11<br><br>1.03 | |
| 52 | 2β-[$N^{\varepsilon}$,$N^{\delta}$-bis (dithiocarbamate)-N-(5-aminopentanoyl)lysinyloxymethyl]-3β-(4'-tolyl)tropane<br>n=4<br>m=4 | 30': cerebellum: 0.114%<br>striatum: 0.133%<br>crossing of blood-brain barrier: 0.358%<br><br>1 h: cerebellum: 0.065%<br>striatum: 0.064%<br>crossing of blood-brain barrier: 0.182% | 1.17<br><br>0.98 | |
| 48 | 2β-[$N^{\varepsilon}$,$N^{\beta}$-bis(dithiocarbamate)(α',δ'-diaminobutyl)-D-lysine]-3β-(4'-tolyl) tropane<br>n=4<br>m=3 | 30': cerebellum: 0.115%<br>striatum: 0.126%<br>crossing of blood-brain barrier: 0.340%<br><br>1 h: cerebellum: 0.050%<br>striatum: 0.050%<br>crossing of blood-brain barrier: 0.143% | 1.09<br><br>1.00 | |
| m and n are data in Table II referring to formula F. | | | | |

Example 33: Synthesis of the bis-dithiocarbamate of adrocorticotropic hormone fragment 1-16 (product (53))

```
Ser-Tyr-Ser-Met-Glu-His-Phe-Arg-Trp-Gly-Lys-Pro-Val-
1               5                   10
Gly-Lys-Lys
    15
```

This hormone fragment of 16 amino acids contains two lysines in positions 15 and 16. We thus prepared the bis-dithiocarbamates from this fragment and according to the process of the present invention and watched how the radiolabelling took place.

0.5 mg of the adrocorticotropic hormone fragment 1-16 is dissolved in 5 ml of injection-grade water. 2 ml of piperidine are added. The mixture is left stirring for 1 hour 30 minutes. The solution is evaporated under vacuum. The residue is taken up in 5 ml of injection-grade water and 3 ml of carbon sulphide are added. The mixture is left stirring for 2 hours. The mixture is then evaporated to dryness. Product (53) consisting of the above hormone fragment comprising the bis-dithiocarbamate according to the present invention on residues 15-16 is thus obtained in freeze-dried form.

Example 34: Radiolabelling of product (53) with technetium

A flask containing a lyophilizate of product (53) is taken up in 1 ml of injection-grade water. 0.5 ml of $TcO^{-}_{4}$ (20 mCi) is added to this solution. After 30 minutes, 0.2 mg of bis-dithiocarbamate (product 53) in a 0.5 M pH 7.4 phosphate buffer and optionally ethanol to dissolve the mixture are added. The manipulation is left to react for 1 hour.

PRC analysis is performed by HPLC. The labelling yield for compound 54 is greater than 95%.

Example 35: Reaction of carbon sulphide with a monoclonal antibody (ACM) (product 55)

5 mg of antibody (anti-ACE) are dissolved in 3 ml of injection-grade water. 400 µl of carbon sulphide are added. The mixture is left stirring at 4°C for 4 hours. After this time, the $CS_2$ is removed under vacuum at room temperature (the volume is brought to 3 ml). Product (55) is stored in solution at -18°C.

Example 36: Radiolabelling of the modified antibody (product 56) with technetium

Synthesis of the TCN intermediate

100 µg of tin chloride, 5 mg of SDH (succinyl dihydrazide) and 5 mg of PDTA (1,2-propanediamino-N,N,N',N'-tetraacetic acid) were freeze-dried in a labelling flask. 3 ml of $TcO^{-}_{4}$ (60 mCi) are added to this lyophilizate. This reagent is left to act for 15 minutes.

1.5 mg of antibody (1 ml) and 1.5 ml of TcN (30 mCi) are placed in a labelling flask. The mixture is left at room temperature for 1 hour. The labelling yield is checked by paper chromatography. Labelling yield for compound (56) is 93%.

Example 37: Formulation of a diagnostic kit

Flask 1: 100 µg of tin chloride, 5 mg of SDH (succinyl dihydrazide) and 5 mg of PDTA (1,2-propane-diamino-N,N,N',N'-tetraacetic acid) were freeze-dried.

Flask 2: 2 mg of bis-dithiocarbamate (more specifically chelation complex according to the invention, containing two dithiocarbamate functions) are packaged in 1 ml of injection-grade water.

Preparation: 3 ml of $TcO_4$ (60 mCi) are added to flask 1. This reagent is left to act for 15 minutes and 1 ml of this solution is added to flask 2. This solution is left to act for one hour. The radiolabelling is ready for injection. Labelling yield ≥95%.

The reaction is analysed by HPLC (reverse-phase, methanol-water).

## Claims

1. Ligand for chelating a metal or a metal complex, **characterized in that** it consists of a bis-dithiocarbamate structure (F) having the following formula:

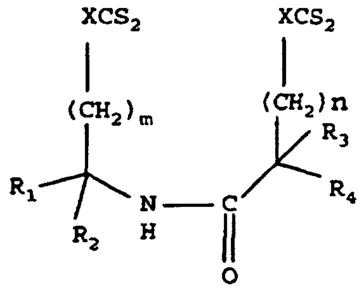

(F)

in which n and m are integers such that 5≤m+n≤10,
X is chosen independently from S and NH,
$R_1$, $R_2$, $R_3$ and $R_4$ are chosen independently from H and an organic function chosen from $-COOR_5$, $NR_5R_6$ and $-CH_2OR_5$ in which $R_5$ and $R_6$, when it is present, are chosen independently from a hydrogen; an amino acid; a peptide; a protein; an organic function; a group chosen from alkoxycarbonyl or aryloxycarbonyl ($-COOR^7$), carboxyl (-COOH), acyloxy ($-O_2R^7$), carbamoyl ($-CONR^7$), cyano (-CN), alkylcarbonyl, alkylarylcarbonyl, arylcarbonyl, arylalkylcarbonyl, hydroxyl (-OH), amino ($NR^7$), halogen, allyl, alkoxy ($-OR^7$), S-alkyl and S-aryl, $R^7$ representing a $C_1$ to $C_{10}$ alkyl or aryl group; an organic molecule chosen from (i) an optionally substituted alkyl, acyl, aryl or alkyne group, (ii) a saturated or unsaturated, optionally substituted or aromatic carbon-based ring or (iii) a saturated or unsaturated, optionally substituted or aromatic heterocycle, these groups and rings (i), (ii) and (iii) possibly being substituted with substituted phenyl groups, substituted aromatic groups or alkoxycarbonyl or aryloxycarbonyl ($-COOR^8$), carboxyl (-COOH), acyloxy ($-O_2R^8$), carbamoyl ($-CONR^8$), alkylcarbonyl, alkylarylcarbonyl, arylcarbonyl, arylalkylcarbonyl, hydroxyl (-OH), amino ($NR^8$), halogen, allyl, alkoxy ($-OR^8$), S-alkyl or S-aryl groups, $R^8$ representing a $C_1$ to $C_{10}$ alkyl or aryl group; a monoclonal antibody; a hormone; and a pharmaceutically acceptable vector.

**2.** Ligand according to Claim 1, in which the bis-dithiocarbamate structure consists of a structure whose formula is chosen from formulae (I), (II), (III) and (IV) below:

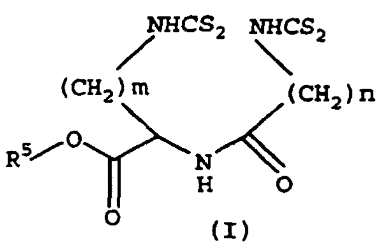

(I)

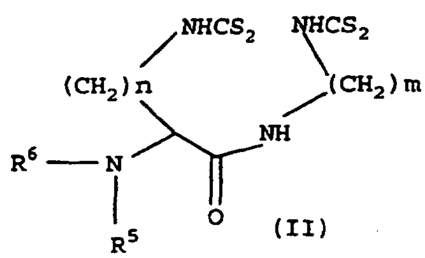

(II)

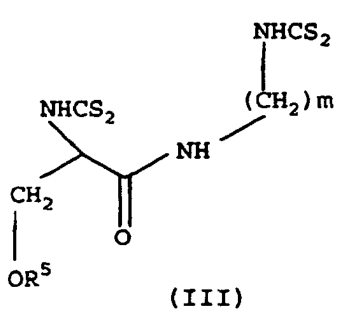

(III)

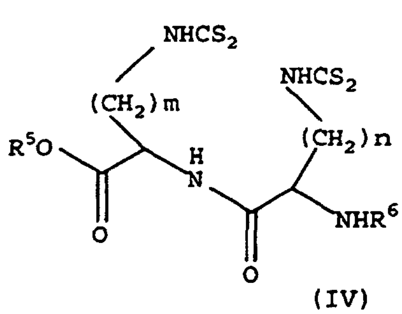

(IV)

in which $R^5$, $R^6$, n and m, when they are present, are as defined in Claim 1.

3. Ligand according to Claim 1 or 2, in which m and n are independently 3, 4 or 5.

4. Ligand according to Claim 2 or 3, in which $R^5$=H.

5. Ligand according to Claim 2 or 3, in which $R^5$=$CH_3$.

6. Ligand according to Claim 2 or 3, in which $R^5$ is a tropane derivative.

7. Ligand according to Claim 2 or 3, in which $R^5$ has the following formula:

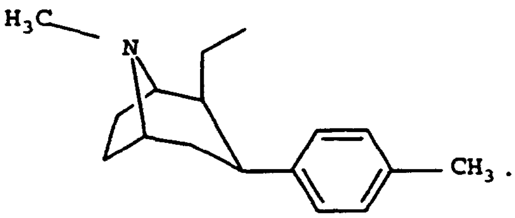

8. Ligand according to Claim 1, consisting of a structure of formula (F) in which $R^1$, $R^3$ and $R^4$ =H and $R^2$ is $NR^5R^6$, in which $R^5$=H and $R^6$ is chosen from:

H or

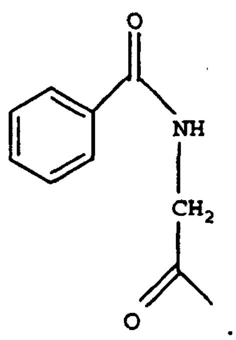

9. **Chelate** consisting of a chelation compound according to any one of Claims 1 to 8 and of a metal or a metal complex.

10. **Chelate** according to Claim 9, in which the metal is copper or an isotope thereof.

11. Chelate according to Claim 9, in which the metal is chosen from a transition metal.

12. Chelate according to Claim 9, in which the metal complex is TcN or ReN.

13. Use of a ligand according to any one of Claims 1 to 8, for manufacturing a medicinal product or a diagnostic product.

14. Use of a ligand according to any one of Claims 1 to 8, for manufacturing a radiopharmaceutical for therapy or for diagnosis.

15. Use of a ligand according to any one of Claims 1 to 8, for manufacturing a radiopharmaceutical for visualizing the uptake of dopamine or serotonin.

16. Use of a chelate according to any one of Claims 9 to 12, for manufacturing a medicinal product or a diagnostic

product.

**17.** Use of a chelate according to any one of Claims 9 to 12, for manufacturing a radiopharmaceutical for therapy or diagnosis.

**18.** Use of a chelate according to any one of Claims 9 to 12, for manufacturing a radiopharmaceutical for visualizing the uptake of dopamine or serotonin.

**19.** Process for manufacturing a bis-dithiocarbamate structure as defined in Claim 1, comprising, successively:

- a step of protecting the XH functions of the compounds of formulae (V) and (VI) below:

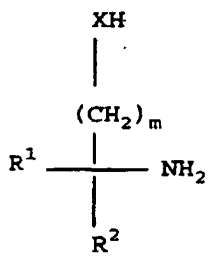

(V)

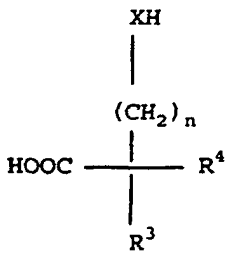

(VI)

in which $R^1$, $R^2$, $R^3$, $R^4$, X, m and n are as defined in Claim 1,

- a step of activating the -COOH function of compound (VI),
- a step of linking the compound of formula (V) and compound (VI) via the activated carboxyl function of compound (VI),
- a step of deprotecting the XH functions, and
- a step of reacting the deprotected XH functions with $CS_2$ to form a bis-dithiocarbamate structure according to Claim 1.

**20.** Process for manufacturing a structure according to Claim 1 or 2, comprising a process according to Claim 19 for manufacturing a bis-dithiocarbamate structure, and also comprising a step of attaching a radical $R^5$ and optionally $R^6$ to this bis-dithiocarbamate structure or to an intermediate product in its manufacture to obtain a chelation compound according to Claim 1 or 2.

**21.** Process for manufacturing a structure as defined in Claim 1 or 2, comprising:

- a step of reacting two ε-$NH_2$ functions of two contiguous amino acids of a precursor molecule of the structure according to Claim 1 or 2 with $CS_2$ so as to form a structure according to Claim 1 or 2,

the precursor molecule constituting the radical $R^5$ and optionally the radical $R^6$.

**22.** Process for preparing a chelation product as defined in Claim 9, comprising the manufacture of a bis-dithiocarbamate structure defined in Claim 1 or 2 according to a manufacturing process defined in Claim 20 or 21, and a reaction for complexing a metal or a metal complex via the said bis-dithiocarbamate structure manufactured.

**23.** Process according to Claim 22, in which the metal is a transition metal.

**24.** Process according to Claim 22, in which the metal is copper.

**25.** Process according to Claim 22, in which the metal complex is TcN or ReN.

**26.** Diagnostic kit comprising a chelating compound according to any one of Claims 1 to 8.

## Patentansprüche

**1.** Ligand zum Chelatisieren eines Metalls oder Metallkomplexes, **dadurch gekennzeichnet, daß** er aus einer Bis (dithiocarbamat)-Struktur F mit der folgenden Formel besteht:

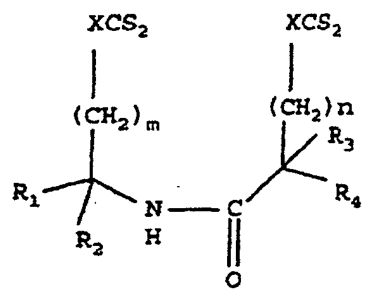

(F)

worin n und m für solche ganzen Zahlen stehen, daß $5 \leq m+n \leq 10$,
X unabhängig unter S und NH ausgewählt ist,
$R_1$, $R_2$, $R_3$ und $R_4$ unabhängig voneinander unter H und einer unter $-COOR_5$, $NR_5R_6$ und $-CH_2OR_5$, worin $R_5$ und $R_6$, sofern vorhanden, unabhängig voneinander unter Wasserstoff, einer Aminosäure, einem Peptid, einem Protein, einer organischen Funktion, einer unter Alkoxycarbonyl oder Aryloxycarbonyl ($-COOR^7-$), Carboxyl (-COOH), Acyloxy ($-O_2R^7$), Carbamoyl ($-CONR^7$), Cyano (-CN), Alkylcarbonyl, Alkylarylcarbonyl, Arylcarbonyl, Arylalkylcarbonyl, Hydroxyl (-OH), Amino ($NR^7$), Halogen, Allyl, Alkoxy ($-OR^7$), S-Alkyl und S-Aryl ausgewählten Gruppe, wobei $R^7$ für eine $C_1$- bis $C_{10}$-Alkyl- oder -Arylgruppe steht, einem unter (i) einer gegebenenfalls substituierten Alkyl-, Acyl-, Aryl- oder Alkingruppe, (ii) einem gesättigten oder ungesättigten, gegebenenfalls substituierten oder aromatischen Ring auf Kohlenstoffbasis oder (iii) einem gesättigten oder ungesättigten, gegebenenfalls substituierten oder aromatischen Heterocyclus ausgewählten organischen Molekül, wobei diese Gruppen und Ringe (i), (ii) und (iii) gegebenenfalls durch substituierte Phenylgruppen, substituierte aromatische Gruppen oder Alkoxycarbonyloder Aryloxycarbonyl- ($-COOR^8-$), Carboxyl- (-COOH), Acyloxy- ($-O_2R^8$), Carbamoyl- ($-CONR^8$), Alkylcarbonyl-, Alkylarylcarbonyl-, Arylcarbonyl-, Arylalkylcarbonyl-, Hydroxyl- (-OH), Amino- ($NR^8$), Halogen-, Allyl-, Alkoxy- ($-OR^8$), S-Alkyl- oder S-Arylgruppen, wobei $R^8$ für eine $C_1$- bis $C_{10}$-Alkyloder -Arylgruppe steht, substituiert sind, einem monoklonalen Antikörper, einem Hormon und einem pharmazeutisch unbedenklichen Vektor ausgewählten organischen Funktion ausgewählt sind, ausgewählt sind.

**2.** Ligand nach Anspruch 1, bei dem die Bis(dithiocarbamat)-Struktur aus einer Struktur besteht, deren Formel unter den nachstehenden Formeln (I), (II), (III) und (IV) ausgewählt ist:

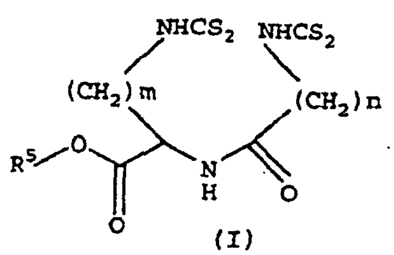

(I)

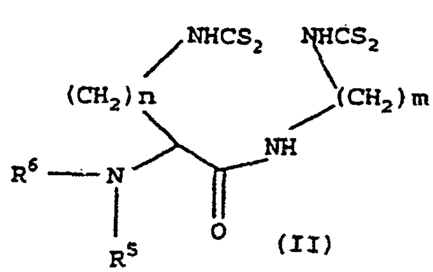

(II)

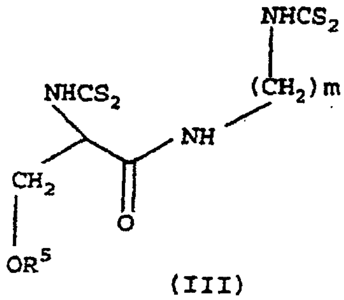

(III)

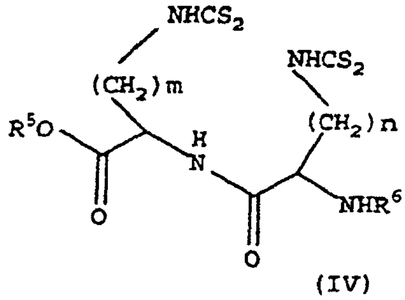

(IV)

worin $R^5$, $R^6$, n und m, sofern vorhanden, die in Anspruch 1 angegebene Bedeutung besitzen.

**3.** Ligand nach Anspruch 1 oder 2, bei dem m und n unabhängig voneinander für 3, 4 oder 5 stehen.

**4.** Ligand nach Anspruch 2 oder 3, in dem $R^5$ = H.

**5.** Ligand nach Anspruch 2 oder 3, in dem $R^5 = CH_3$.

**6.** Ligand nach Anspruch 2 oder 3, in dem $R^5$ für ein Tropanderivat steht.

**7.** Ligand nach Anspruch 2 oder 3, in dem $R^5$ die folgende Formel hat:

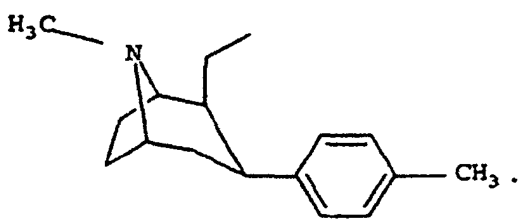

**8.** Ligand nach Anspruch 1, bestehend aus einer Struktur der Formel (F), worin $R^1$, $R^3$ und $R^4$ = H und $R^2$ für $NR^5R^6$, worin $R^5$ = H und $R^6$ unter:

H oder

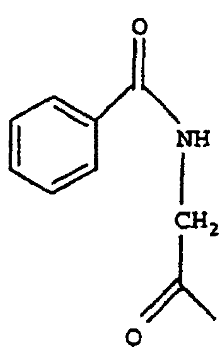

ausgewählt ist, steht.

**9.** Chelat, bestehend aus einem Chelatbildner nach einem der Ansprüche 1 bis 8 und einem Metall oder einem Metallkomplex.

**10.** Chelat nach Anspruch 9, bei dem es sich bei dem Metall um Kupfer oder ein Isotop davon handelt.

**11.** Chelat nach Anspruch 9, bei dem das Metall unter Übergangsmetallen ausgewählt ist.

**12.** Chelat nach Anspruch 9, bei dem es sich bei dem Metallkomplex um TcN oder ReN handelt.

**13.** Verwendung eines Liganden nach einem der Ansprüche 1 bis 8 zur Herstellung eines Arzneimittels oder eines Diagnostikprodukts.

**14.** Verwendung eines Liganden nach einem der Ansprüche 1 bis 8 zur Herstellung eines Radiopharmazeutikums für die Therapie oder Diagnose.

**15.** Verwendung eines Liganden nach einem der Ansprüche 1 bis 8 zur Herstellung eines Radiopharmazeutikums zur Visualisierung der Aufnahme von Dopamin oder Serotonin.

**16.** Verwendung eines Chelats nach einem der Ansprüche 9 bis 12 zur Herstellung eines Arzneimittels oder eines Diagnostikprodukts.

**17.** Verwendung eines Chelats nach einem der Ansprüche 9 bis 12 zur Herstellung eines Radiopharmazeutikums für die Therapie oder Diagnose.

**18.** Verwendung eines Chelats nach einem der Ansprüche 9 bis 12 zur Herstellung eines Radiopharmazeutikums zur Visualisierung der Aufnahme von Dopamin oder Serotonin.

**19.** Verfahren zur Herstellung einer Bis(dithiocarbamat)-Struktur gemäß Anspruch 1, bei dem man nacheinander:

- die XH-Funktionen der nachstehenden Verbindungen (V) und (VI):

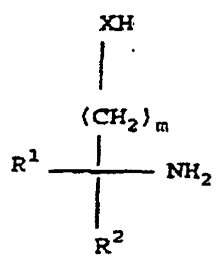

(V)

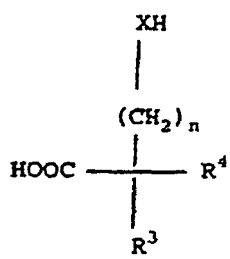

(VI)

  worin $R^1$, $R^2$, $R^3$, $R^4$, X, m und n die in Anspruch 1 angegebene Bedeutung besitzen, schützt,
- die -COOH-Funktion der Verbindung (VI) aktiviert,
- die Verbindung der Formel (V) und die Verbindung (VI) über die aktivierte Carboxylfunktion der Verbindung (VI) verknüpft,
- die XH-Funktionen entschützt und
- die entschützten XH-Funktionen mit $CS_2$ umsetzt, wobei man die Bis(dithiocarbamat)-Struktur nach Anspruch 1 erhält.

**20.** Verfahren zur Herstellung einer Struktur nach Anspruch 1 oder 2 mit einem Verfahren nach Anspruch 19 zur Herstellung einer Bis(dithiocarbamat)-Struktur, bei dem man ferner an diese Bis(dithiocarbamat)-Struktur oder ein Zwischenprodukt bei deren Herstellung einen Rest $R^5$ und gegebenenfalls $R^6$ anbringt, wobei man einen Chelatbildner nach Anspruch 1 oder 2 erhält.

**21.** Verfahren zur Herstellung einer Struktur gemäß Anspruch 1 oder 2, bei dem man:

- zwei ε-$NH_2$-Funktionen von zwei zusammenhängenden Aminosäuren eines Vorläufermoleküls der Struktur nach Anspruch 1 oder 2 mit $CS_2$ umsetzt, wobei man eine Struktur nach Anspruch 1 oder 2 erhält,

wobei das Vorläufermolekül den Rest $R^5$ und gegebenenfalls $R^6$ umfaßt.

**22.** Verfahren zur Herstellung eines Chelatisierungsprodukts nach Anspruch 9, bei dem man nach einem Herstellungsverfahren gemäß Anspruch 20 oder 21 eine Bis(dithiocarbamat)-Struktur gemäß Anspruch 1 oder 2 herstellt und eine Reaktion zur Komplexierung eines Metalls oder Metallkomplexes mit der hergestellten Bis(dithiocarbamat)-Struktur durchführt.

**23.** Verfahren nach Anspruch 22, bei dem es sich bei dem Metall um ein Übergangsmetall handelt.

**24.** Verfahren nach Anspruch 22, bei dem es sich bei dem Metall um Kupfer handelt.

**25.** Verfahren nach Anspruch 22, bei dem es sich bei dem Metallkomplex um TcN oder ReN handelt.

**26.** Diagonstik-Kit, enthaltend einen Chelatbildner nach einem der Ansprüche 1 bis 8.

**Revendications**

**1.** Ligand de chélation d'un métal ou d'un complexe de métal, **caractérisé en ce qu'**il est constitué d'une structure bis dithiocarbamate (F) de formule suivante :

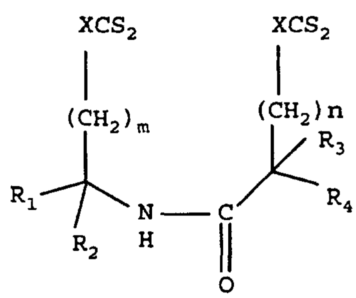

(F)

dans laquelle n et m sont des nombres entiers tels que $5 \leq m+n \leq 10$,
X est choisi indépendamment parmi S ou NH,
$R_1$, $R_2$, $R_3$ et $R_4$ sont choisis indépendamment parmi H ou une fonction organique choisie parmi $-COOR_5$, $NR_5R_6$, $-CH_2OR_5$ dans laquelle $R_5$ et $R_6$ lorsqu'il est présent sont choisis indépendamment parmi un hydrogène ; un acide aminé ; un peptide ; une protéine ; une fonction organique ; un groupe choisi parmi alcoxycarbonyle ou aryloxycarbonyle ($-COOR^7$), carboxy, (-COOH), acyloxy ($-O_2R^7$), carbamoyle ($-CONR^7$), cyano (-CN), alkylcarbonyle, alkylarylcarbonyle, arylcarbonyle, arylalkylcarbonyle, hydroxy (-OH), amino, ($NR^7$), halogène, allyle, alcoxy ($-OR^7$), S-alkyle, S-aryle, $R^7$ représentant un groupe alkyle ou aryle en $C_1$ à $C_{10}$; une molécule organique choisie parmi un groupe (i) alkyle, acyle, aryle ou alcyne éventuellement substitué, ou un cycle (ii) carboné, saturé ou non, éventuellement substitué ou aromatique, ou un hétérocycle (iii), saturé ou non, éventuellement substitué ou aromatique, ces groupes et cycles (i), (ii) et (iii) pouvant être substitués par des groupes phényles substitués, des groupes aromatiques substitués ou des groupes alcoxycarbonyle ou aryloxycarbonyle ($-COOR^8$), carboxy (-COOH), acyloxy ($-O_2R^8$), carbamoyle ($-CONR^8$), alkylcarbonyle, alkylarylcarbonyle, arylcarbonyle, arylalkylcarbonyle, hydroxy (-OH), amino ($NR^8$), halogène, allyle, alcoxy ($-OR^8$), S-alkyle, S-aryle, $R^8$ représentant un groupe alkyle ou aryle en $C_1$ à $C_{10}$; un anticorps monoclonal ; une hormone; et un vecteur pharmaceutiquement acceptable.

**2.** Ligand selon la revendication 1, dans lequel la structure bis dithiocarbamate est constituée d'une structure de

formule choisie parmi les formules (I), (II), (III), (IV) suivantes :

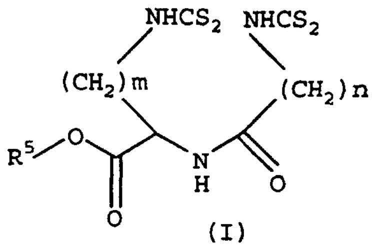

(I)

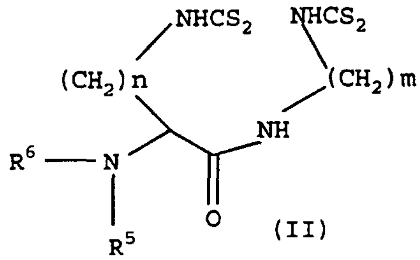

(II)

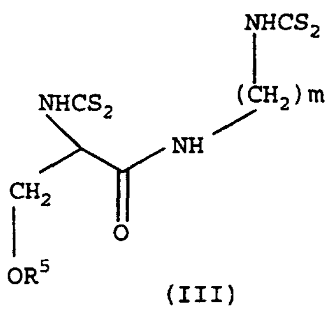

(III)

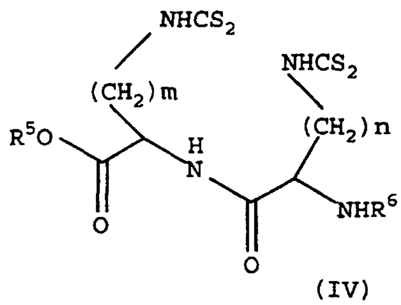

(IV)

dans lesquelles $R^5$, $R^6$, n et m lorsqu'ils sont présents, sont tels que définis dans la revendication 1.

**3.** Ligand selon la revendication 1 ou 2, dans lequel m et n sont indépendamment 3, 4 ou 5.

**4.** Ligand selon la revendication 2 ou 3, dans lequel $R^5$=H.

**5.** Ligand selon la revendication 2 ou 3, dans lequel $R^5$=$CH_3$.

**6.** Ligand selon la revendication 2 ou 3, dans lequel $R^5$ est un dérivé du tropane.

**7.** Ligand selon la revendication 2 ou 3, dans lequel $R^5$ est de formule suivante :

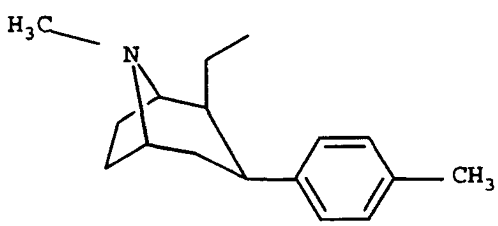

**8.** Ligand selon la revendication 1, constitué d'une structure de formule (F) dans laquelle $R^1$, $R^3$ et $R^4$ =H et $R^2$ est $NR^5R^6$, avec $R^5$=H et $R^6$ est choisi parmi :

H ou

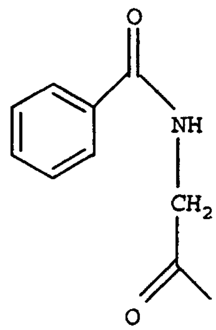

**9.** Chélate constitué d'un ligand selon l'une quelconque des revendications 1 à 8 et d'un métal ou d'un complexe de métal.

**10.** Chélate selon la revendication 9, dans lequel le métal est du cuivre ou l'un de ses isotopes.

**11.** Chélate selon la revendication 9, dans lequel le métal est choisi parmi un métal de transition.

**12.** Chélate selon la revendication 9, dans lequel le complexe de métal est du TcN ou du ReN.

**13.** Utilisation d'un ligand selon l'une quelconque des revendications 1 à 8 pour fabriquer un médicament ou un produit pour le diagnostic.

**14.** Utilisation d'un ligand selon l'une quelconque des revendications 1 à 8 pour fabriquer un radiopharmaceutique pour la thérapie ou pour le diagnostic.

**15.** Utilisation d'un ligand selon l'une quelconque des revendications 1 à 8 pour fabriquer un radiopharmaceutique pour visualiser la recapture de la dopamine ou de la sérotonine.

**16.** Utilisation d'un chélate selon l'une quelconque des revendications 9 à 12 pour fabriquer un médicament ou un produit pour le diagnostic.

**17.** Utilisation d'un chélate selon l'une quelconque des revendications 9 à 12 pour fabriquer un radiopharmaceutique pour la thérapie ou le diagnostic.

**18.** Utilisation d'un chélate selon l'une quelconque des revendications 9 à 12 pour fabriquer un radiopharmaceutique pour visualiser la recapture de la dopamine ou de la sérotonine.

**19.** Procédé de fabrication d'une structure bis dithiocarbamate telle que définie dans la revendication 1 comprenant successivement :

- une étape de protection des fonctions XH des composés de formules (V) et (VI) suivantes :

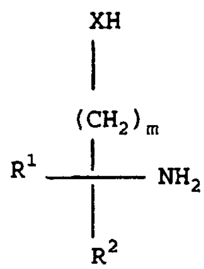

(V)

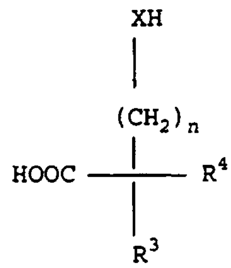

(VI)

dans lesquelles $R^1$, $R^2$, $R^3$, $R^4$, X, m et n sont tels que définis dans la revendication 1,

- une étape d'activation de la fonction -COOH du composé (VI),
- une étape de liaison du composé de formule (V) et du composé (VI) par la fonction carboxyle activée du composé (VI),
- une étape de déprotection des fonctions XH, et
- une étape de réaction des fonctions XH déprotégées avec du $CS_2$ pour former une structure bis dithiocarbamate selon la revendication 1.

**20.** Procédé de fabrication d'une structure selon la revendication 1 ou 2 comprenant un procédé selon la revendication 19 de fabrication d'une structure bis dithiocarbamate, et comprenant en outre une étape de fixation d'un radical $R^5$ et éventuellement $R^6$ sur cette structure bis dithiocarbamate ou sur un produit intermédiaire de sa fabrication pour obtenir un ligand selon la revendication 1 ou 2.

**21.** Procédé de fabrication d'une structure telle que définie dans la revendication 1 ou 2, comprenant :

- une étape de réaction de deux fonctions ε-$NH_2$ de deux acides aminés contigus d'une molécule précurseur de la structure selon la revendication 1 ou 2 avec du $CS_2$ de manière à former une structure selon la revendication 1 ou 2,

la molécule précurseur constituant le radical $R^5$ et éventuellement le radical $R^6$.

**22.** Procédé de préparation d'un chélate tel que défini dans la revendication 9 comprenant la fabrication d'une structure bis dithiocarbamate définie dans la revendication 1 ou 2 suivant un procédé de fabrication défini dans la revendication 20 ou 21, et une réaction de complexation d'un métal ou d'un complexe de métal par ladite structure bis dithiocarbamate fabriquée.

**23.** Procédé selon la revendication 22 dans lequel le métal est un métal de transition.

**24.** Procédé selon la revendication 22 dans lequel le métal est du cuivre.

**25.** Procédé selon la revendication 22 dans lequel le complexe de métal est du TcN ou du ReN.

**26.** Trousse de diagnostic comprenant un ligand selon l'une quelconque des revendications 1 à 8.